# EUROPEAN PATENT SPECIFICATION

(11) **EP 3 937 927 B1**
(45) Date of publication and mention of the grant of the patent: **17.04.2024**
(21) Application number: 20710518.0
(22) Date of filing: 13.03.2020
(51) Int. Cl.: A61K 31/353, A61K 31/4415, A61K 31/593, A61K 31/235, A61K 31/355, A61P 35/00

(54) **TREATMENT OF FIBROIDS WITH VITAMIN D AND AN AGENT SUCH AS EPIGALLOCATECHIN GALLATE (EGCG)**
BEHANDLUNG VON FIBROIDEN MIT VITAMIN D UND EINEM MITTEL WIE ETWA EPIGALLOCATECHINGALLAT (EGCG)
TRAITEMENT DE LÉIOMYOMES AVEC DE LA VITAMINE D ET UN AGENT TEL QUE LE GALLATE D'ÉPIGALLOCATÉCHINE (EGCG)

(30) Priority: 15.03.2019 IT 201900003843
(43) Date of publication of application: 19.01.2022
(73) Proprietor: LO.LI. PHARMA S.R.L., 00156 Roma (IT)
(72) Inventor: UNFER, Vittorio, 00156 Rome (RM) (IT)
(74) Representative: Pace Napoleone, Maria
(86) International application number: PCT/EP2020/056799
(87) International publication number: WO 2020/187729

(56) References cited:
- US-B1- 8 221 803
- MICHAL CIEBIERA ET AL: "Alternative Oral Agents in Prophylaxis and Therapy of Uterine Fibroids-An Up-to-Date Review", INTERNATIONAL JOURNAL OF MOLECULAR SCIENCES, vol. 18, no. 12, 1 December 2017 (2017-12-01), page 2586, XP055641506, DOI: 10.3390/ijms18122586
- CHEETHAM PHILIPPA J: "Role of Complimentary Therapy for Male LUTS", CURRENT UROLOGY REPORTS, CURRENT SCIENCE, US, vol. 14, no. 6, 13 September 2013 (2013-09-13), pages 606-613, XP035375508, ISSN: 1527-2737, DOI: 10.1007/S11934-013-0372-3 [retrieved on 2013-09-13]

## Description

### Field of the invention

The present invention relates to Vitamin D in combination with one or more agents selected from the group consisting of: epigallocatechin gallate (EGCG), catechin, gallocatechin and epicatechin gallate (ECG), preferably selected from epigallocatechin-3-gallate, (-)-epigallocatechin-3-gallate, (-)-gallocatechin-3-O-gallate (GCG), (-)-gallocatechin-3-gallate, (+)-catechin (C), (-)-epicatechin (EC), (+)-gallocatechin (GC), (-)- epigallocatechin (EGC), (-)-epicatechin-3-gallate, (-)-catechin-3-gallate and (+)-catechin-3-gallate, for the prevention, control and/or treatment of a fibroid selected from a uterine fibroid or a fibroadenoma in a subject.

### Background of the invention

Uterine fibroids, also known as uterine leiomyomas or myomas, are the most common tumors of the female genital tract. They are monoclonal benign tumors of uterine smooth muscle, thus originating from the myometrium that develop in women of childbearing age, mainly between the ages of 35 and 50 years. Uterine fibroids are well known estrogen-dependent tumors in the uterus of premenopausal women. A hallmark of uterine leiomyoma is the excessive deposition of extracellular matrix (ECM) [Malik M, et al. Matrix Biol 2012, 7-8:389-397]. The ECM components contributing to the majority of the volume in uterine fibroids are fibronectin, collagen and proteoglycan [Ingber D and Folkman J. J Cell Biol 1989, 109:317-330]. As with other tissues in the body, the ECM action in uterine fibroids stimulates cell-cell adhesion, cell migration, cell proliferation and cell differentiation and promotes further ECM deposition [Ingber D and Folkman J. J Cell Biol 1989, 109:317-330]. The ECM in uterine tissue, whether in normal myometrium or in a fibroid, contains a substantial amount of collagen. The excess of ECM in uterine fibroids is due to both an overproduction of collagen and a decrease in collagen breakdown, and the collagen deposition is a greater proportion of the ECM composition in uterine fibroids compared to healthy uterine tissue [Leppert PC, et al. Fertil Steril 2004, 82:1182-1187]. The increased deposition of collagen and fibronectin is partly responsible for the changes in the physical characteristics of leiomyoma tissue compared to that of healthy myometrium. The orientation and organization of collagen in uterine fibroids interstitial matrix is abnormal, forming their rigid characteristic [Tomasek JJ, et al. Nat Rev Mol Cell Biol 2002, 3:349-363]. Smooth muscle cells of the myometrium could react to injury with the synthesis of extracellular fibrous matrix.

Uterine fibroids often develop asymptomatically. They may also be associated with different symptoms, such as: excessive menstrual bleeding (menorrhagia), dysmenorrhoea and intermenstrual bleeding, chronic pelvic pain, compression of the surrounding organs, painful sexual intercourse, anemia and pressure symptoms such as a sensation of bloatedness, increased urinary frequency, and bowel disturbance. In addition, they may compromise reproductive functions, possibly contributing to subfertility, early pregnancy loss, later pregnancy complications, infertility, recurrent abortion, preterm delivery.

The effect of uterine fibroids on the quality of life and the overall cost of treatment are significant concerns worldwide.

Other fibroid conditions include fibroadenoma and prostatic hyperplasia. Fibroadenoma, which is a benign human breast tumor, is characterized by proliferation of epithelial and fibroblast stromal components of the terminal ductal unit.

Prostatic hyperplasia, also known as benign prostatic hyperplasia (BPH), prostatic enlargement or prostatic leiomyoma, is a noncancerous growth in size of the prostate gland with an increased fibrillar collagen or extracellular matrix deposition. Therefore, there is still the need to provide a non-invasive and effective treatment for fibroids.

The US patent US 8 221 803 B1, discloses the treatment of BPH with a combination comprising *inter alia* vitamin D3, ECG (e.g. from green tea extract) and alpha-tocopherol. Ciebiera M. et al. (Int. J. of Mol. Sci, 2017:18(12), 2586) is a review on the oral agents available in the prophylaxis and therapy of uterine fibroids. Both vitamin D and EGCG are mentioned among other compounds as valuable alternatives.

### EGCG

Epigallocatechin gallate (EGCG) is an ester of epigallocatechin and gallic acid, extracted from green tea. Its effect in oncology has been studied thoroughly and has presented beneficial effects. EGCG inhibits proliferation, induces apoptosis in human uterine fibroid cells and reduces the volume of uterine fibroid tumors in mice [Zhang, D. et al., Am. J. Obstet. Gynecol. 2010, 202, 289.e1-289.e9]. One clinical study showed a reduction of 32.6% in the total fibroid volume in women with uterine fibroids after treatment with EGCG [E. Roshdy et al, Int. J. Women's Health, 2013:5 477-486]. In those studies, EGCG was a modulation agent in the proliferation, transformation, and inflammation in uterine fibroid tumors. EGCG exerts its action possibly modulating different factors involved in proliferation, cell cycle regulation and apoptosis such as proliferating cell nuclear antigen (PCNA), cyclin-dependent kinase 1 (CDK1) and B-cell lymphoma 2 (BCL2).

### Vitamins D

Vitamins D are a group of fat-soluble secosteroids which exert different biological activities. Their role in increasing intestinal absorption of calcium, magnesium, and phosphate is well known. Indeed, Vitamins D modify the activity of bone cells and are important for the formation of new bone in children and adults. Vitamins D are mostly produced in the skin from sunlight and about 10% is also absorbed from dietary intake. The liver and kidneys convert vitamins D, into the active hormone, which is called calcitriol.

It has been demonstrated that 1,25-dihydroxyvitamin D inhibits uterine fibroids cell proliferation in vitro and decreases the size of uterine fibroids in animal models. Vitamin D (vitamin D3) reduces uterine fibroids cell proliferation *in vitro,* and uterine fibroid tumor growth in *in vivo* animal models. According to *in vitro* experiments, uterine fibroid cells are highly sensitive to the growth-inhibiting effect of the active form of vitamin D. Vitamin D regulates the cellular proliferation and induction of apoptosis through the downregulation of proliferating cell nuclear antigen (PCNA) and cyclin-dependent kinase 1 (CDK1).

Vitamin D deficiency is associated with an increased risk of uterine fibroids and in particular there is an inverse relationship between vitamin D serum levels and the severity of fibroids.

### Summary of the invention

The author has surprisingly found that the use of Vitamin D in combination with at least one agent selected from the group consisting of: epigallocatechin gallate (EGCG), catechin, gallocatechin and epicatechin gallate (ECG) has robust inhibitory effects on fibronectin and collagen release in fibroblast cells *in vitro.* A combination of Vitamin D and said at least one agent exerts a synergic action, reducing the proliferation of fibrotic components and then reducing the excessive deposition of extracellular matrix common in fibroids. The increased efficacy of Vitamin D in combination with said at least one agent has a major effect on the reduction of proliferation of fibrotic components compared to the single effect of vitamin D alone or to said at least one agent alone.

In other words, the author has surprisingly found that the administration of a combination or association of Vitamin D and at least one agent selected from the group consisting of: EGCG, catechin, gallocatechin and ECG exerts more advantageous effects than those of single components for treating fibroblast cells. Up to now, it was known that administration of either Vitamin D or EGCG could be used to treat uterine fibroids, but it was not known or suggested that such compounds together could be more advantageous in the treatment of this fibroid condition. Evidence of the reduction of the volume of fibroid represents an absolutely novel effect advantageously achievable using Vitamin D and at least one agent selected from the group consisting of: EGCG, catechin, gallocatechin and ECG.

Therefore, an object of the present invention is vitamin D in combination with one or more agent selected from the group consisting of: epigallocatechin gallate (EGCG), catechin, gallocatechin and epicatechin gallate (ECG), preferably selected from epigallocatechin-3-gallate, (-)-epigallocatechin-3-gallate, (-)-gallocatechin-3-O-gallate (GCG), (-)-gallocatechin-3-gallate, (+)-catechin (C), (-)-epicatechin (EC), (+)-gallocatechin (GC), (-)-epigallocatechin (EGC), (-)-epicatechin-3-gallate, (-)-catechin-3-gallate and (+)-catechin-3-gallate, for use in the prevention, control and/or treatment of a fibroid selected from a uterine fibroid or a fibroadenoma. Preferably, said one or more agent is epigallocatechin gallate (EGCG). Preferably, said vitamin D is selected from the group consisting of: Vitamin D3 (cholecalciferol), Vitamin D2 (ergocalciferol), 25(OH)vitamin D (calcidiol), 1,25 dihydroxy-vitamin D (calcitriol) and combinations thereof. Preferably, vitamin D refers to vitamin D3. Preferably, said fibroid is a uterine fibroid. Preferably, said uterine fibroid is intramural, subserosal and/or submucosal.

Preferably, vitamin D is in an amount of approximately from 1 µg to 100 µg per day.

Preferably, vitamin D is in an amount of approximately from 25 µg to 50 µg per day, preferably of approximately 50 µg per day. Preferably, vitamin D is administered twice per day, each administration being of approximately 25 µg. Preferably, 1 IU of vitamin D corresponds to 0.025 µg of vitamin D such that vitamin D is in an amount of approximately from 40 IU to 4000 IU.

Preferably, said one or more agent is in an amount of approximately from 5 mg to 1000 mg per day. Preferably, said one or more agent is in an amount of approximately 300 mg per day. Preferably, said one or more agent is administered twice per day, each administration being of approximately 120 mg to 180 mg, preferably of approximately 150 mg. It is to be understood that, when more agents are present, the amounts of the one or more agent may refer to those of each agent or to the sum of all the agents present.

Preferably, vitamin D and said one or more agent are in a weight ratio of approximately from 1 : 50 to 1 : 1000000. Preferably, vitamin D and said one or more agent are in a weight ratio of approximately from 1 : 100 to 1 : 1000, preferably vitamin D and said one or more agent are in a weight ratio of approximately from 1 : 300 to 1 : 600. Preferably, vitamin D and said one or more agent are in a weight ratio of approximately 1 : 6000, 1 : 50, 1 : 2000, 1 : 2500, 1 : 3000, 1 : 5000, 1 : 7000, 1 : 10000, or 1 : 1000000. It is to be understood that, when more agents are present, the parts in weight of the one or more agent may refer to those of each agent or to the sum of all the agents present.

In a preferred aspect, vitamin D and the one or more agent as defined above are for use with at least one further agent, optionally selected from the group consisting of: vitamin B, a polyphenol and DL-alpha-tocopherol, preferably said at least one further agent is vitamin B6. Then, the present invention also provides vitamin D, the one or more agent as defined above and at least one further agent optionally selected from the group consisting of: vitamin B, a polyphenol and DL-alpha-tocopherol, preferably said at least one further agent is vitamin B6, for use as defined above.

Preferably vitamin D, said one or more agent and said at least one further agent are in a weight ratio of approximately from 1 : 5000 : 100 to 1 : 10000 : 1000. Preferably, vitamin D, said one or more agent and said at least one further agent are in a weight ratio of approximately from 1 : 5000 : 1000 to 1 : 10000 : 100. Preferably, vitamin D, said one or more agent and said at least one further agent are in a weight ratio of approximately 1 : 6000 : 200, 1 : 5000 : 1000, 1 : 10000 : 100 or 1 : 10000 : 1000. Preferably, said at least one further agent is in an amount of approximately 1 to 100 mg per day, preferably approximately 1 to 10 mg per day, preferably approximately 1 to 5 mg per day, preferably approximately 1, 5 or 10 mg per day.

Preferably, said further agent is vitamin B6. Preferably, said vitamin B6 is in an amount of approximately from 1 to 100 mg per day, preferably approximately 1 to 10 mg per day, preferably approximately 1 to 5 mg per day, preferably approximately 1, 5 or 10 mg per day. Preferably, vitamin B6 is administered twice per day, each administration being of approximately 5 mg.

In a preferred embodiment, the present invention provides vitamin D and the one or more agent for use as defined above, wherein said one or more agent is epigallocatechin gallate (EGCG) and/or wherein said fibroid is a uterine fibroid. Then, in a preferred embodiment, the present invention provides vitamin D and epigallocatechin gallate (EGCG) for use in the treatment of a uterine fibroid.

Preferably, said one or more agent is in the form of a tea plant leaf or of an extract of a tea plant leaf. Preferably, said tea plant leaf is a green tea plant leaf. Preferably, said tea plant leaf comprises said one or more agent in an amount of approximately from 30 wt% to 95 wt%, preferably approximately 30 wt%, 45 wt%, 50 wt% or 95 wt%. It is to be understood that, when more agents are present, the wt% of the one or more agent in said leaf may refer to that of each agent or to the sum of all the agents present. In a preferred embodiment of the above-described use, said vitamin D and said one or more agent reduce the release of fibronectin and/or collagen.

The combination according to the present invention comprising vitamin D and one or more agent as defined above, has been shown to reduce the release of fibronectin and/or collagen.

Preferably, reducing the release of fibronectin and/or collagen is measured by comparison to a suitable control as no treatment at all, treatment with vitamin D alone or treatment with the one or more agent alone.

In a preferred embodiment of the above-described use, said vitamin D and said one or more agent reduce fibroid volume.

The combination according to the present invention comprising vitamin D and one or more agent as defined above, has been shown to reduce fibroid volume.

Preferably, reducing the fibroid volume is measured by comparison to a suitable control as no treatment at all, treatment with vitamin D alone or treatment with the one or more agent alone.

Preferably, said fibroid is a uterine fibroid and said prevention, control and/or treatment of a uterine fibroid comprises the prevention, control and/or treatment of at least one symptom of a uterine fibroid. Preferably, said symptom of a uterine fibroid is selected from the group consisting of: excessive menstrual bleeding (menorrhagia), dysmenorrhea, intermenstrual bleeding, pelvic pain (acute or chronic), painful sexual intercourse, anemia, a compromised reproductive function, subfertility, infertility, early pregnancy loss, recurrent abortion, preterm delivery and a pregnancy complication, fatigue and a pressure symptom such as bloatedness, increased urinary frequency, bowel disturbance.

Preferably, vitamin D and said one or more agent are administered simultaneously and/or orally and/or twice a day.

Preferably, vitamin D and said one or more agent are for use in combination with a further therapeutic intervention. Preferably said further therapeutic intervention is selected from the group consisting of: surgery, a gonadotropin-releasing hormone agonist (GnRHa), a selective progesterone receptor modulator (sPRMs), an anti-hormonal agent, a hormone, an anti-inflammatory painkiller and tranexamic acid. Preferably, said surgery is selected from the group consisting of: conservative surgical therapy (myomectomy), hysterectomy, myolysis, uterine artery embolization (UAE) and magnetic resonance imaging-guided focused ultrasound surgery (MRgFUS). Preferably, said hormone is an estroprogestin or a progesterone. Preferably, said anti-inflammatory painkiller is a nonsteroidal anti-inflammatory drug (NSAIDs). Preferably, said a selective progesterone receptor modulator is ulipristal acetate (UPA).

It is also an object of the present invention a composition comprising:
- vitamin D,
- one or more agent selected from the group consisting of: epigallocatechin gallate (EGCG), catechin, gallocatechin and epicatechin gallate (ECG), preferably selected from epigallocatechin-3-gallate, (-)-epigallocatechin-3-gallate, (-)-gallocatechin-3-O-gallate (GCG), (-)-gallocatechin-3-gallate, (+)-catechin (C), (-)-epicatechin (EC), (+)-gallocatechin (GC), (-)-epigallocatechin (EGC), (-)-epicatechin-3-gallate, (-)-catechin-3-gallate and (+)-catechin-3-gallate,
- an excipient or a diluent, and
- optionally at least one further agent.

It is to be understood that any amount, weight ratio, wt %, definition of the one or more agent, definition of the further agent, definition of the fibroid described above also applies to the composition.

Said composition is for use in the prevention, control and/or treatment of a fibroid selected from a uterine fibroid or a fibroadenoma.

Preferably, in the composition, said one or more agent is selected from the group consisting of: epigallocatechin-3-gallate, (-)-epigallocatechin-3-gallate, (-)-gallocatechin-3-O-gallate (GCG), (-)-gallocatechin-3-gallate, (+)-catechin (C), (-)-epicatechin (EC), (+)-gallocatechin (GC), (-)-epigallocatechin, (-)-epicatechin-3-gallate, (-)-catechin-3-gallate and (+)-catechin-3-gallate. Preferably, said one or more agent in the composition is one agent being epigallocatechin gallate (EGCG). Preferably, said vitamin D is selected from the group as defined above.

Preferably, in the composition, said one or more agent is in the form of a tea plant leaf or of an extract of a tea plant leaf as defined above. Preferably, vitamin D in the composition is in an amount of approximately from 1 µg to 100 µg, preferably of approximately 25 µg or 50 µg. Preferably, said one or more agent in the composition is in an amount of approximately from 5 mg to 1000 mg, preferably approximately 300 mg or 120 mg to 180 mg, preferably of approximately 150 mg. Preferably, in said composition, vitamin D and said one or more agent are in a weight ratio of approximately from 1 : 50 to 1 : 1000000, preferably of approximately 1 : 6000, 1 : 50, 1 : 2000, 1 : 2500, 1 : 3000, 1 : 5000, 1 : 7000, 1 : 10000, or 1 : 1000000.

Preferably, said excipient and/or diluent is selected from the group consisting of: calcium phosphate, dicalcium phosphate, microcrystalline cellulose, magnesium stearate, silicon dioxide, sucrose, gum arabic, corn starch, medium-chain triglycerides, tricalcium phosphate, cross-linked sodium carboxymethylcellulose, hydroxypropyl methylcellulose, polyethylene glycol, titanium dioxide, polyvinylpyrrolidone, talc, erythritol, xylitol, steviol glycosides and sucralose.

Preferably, said at least one optional further agent in the composition is selected from the group consisting of: vitamin B, a polyphenol and DL-alpha-tocopherol. Preferably, in said composition vitamin D, said one or more agent and said at least one further optional agent are in a weight ratio of approximately from 1 : 5000 : 100 to 1 : 10000 : 1000, preferably of approximately 1 : 6000 : 200, 1 : 5000 : 1000, 1 : 10000 : 100 or 1 : 10000 : 1000. Preferably, said at least one optional further agent in the composition is in an amount of approximately 1 to 100 mg per day, preferably approximately 1 to 10 mg per day, preferably approximately 1 to 5 mg per day, preferably approximately 1, 5 or 10 mg per day. Preferably, said at least one optional further agent in the composition is vitamin B6, preferably in an amount of approximately from 1 to 100 mg, preferably approximately 1 to 10 mg, preferably approximately 1 to 5 mg, preferably approximately 1, 5 or 10 mg per day.

Preferably, said composition is administered orally. Preferably, said composition is administered once or twice per day.

Preferably, said composition is in the form of a tablet, a hard capsule, a soft gel capsule, a powder, a syrup, a cachet, a troche, a lozenge or a pastille.

Preferably, said composition is for use in combination with a further therapeutic intervention. Preferably said further therapeutic intervention is selected from the group consisting of: surgery, a gonadotropin-releasing hormone agonist (GnRHa), a selective progesterone receptor modulator (sPRMs), an anti-hormonal agent, a hormone, an anti-inflammatory painkiller and tranexamic acid, all of which may preferably be as defined above.

Preferably, said composition is a pharmaceutical composition, a supplement, a food product or a drink product. Then, the present invention also provides a pharmaceutical composition, a supplement, a food product or a drink product comprising the components defined above for the composition.

In case the composition is a pharmaceutical composition, said excipient and/or diluent is preferably pharmaceutically acceptable.

Preferably, said supplement is any type of dietary supplement. Preferably, said food product is an edible bar or an edible snack.

### Description of the figures

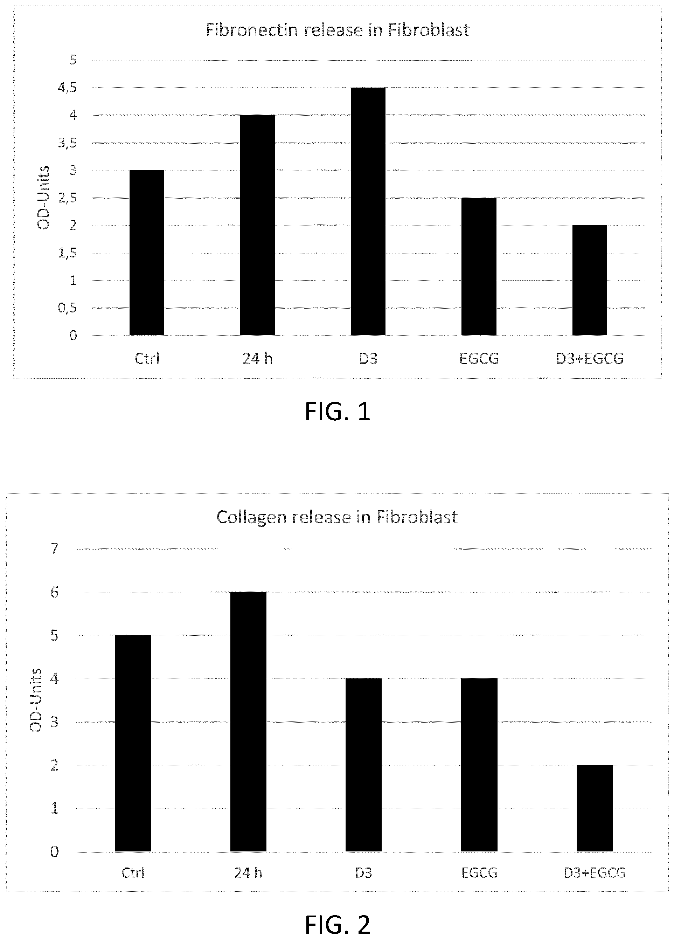

Fig. 1 and 2 respectively show fibronectin and collagen release in cultured fibroblasts. Ctrl = fibroblasts after 18 days of culture (no TGF-β1 stimulation and no treatment); 24 h = fibroblasts after 18 days of culture followed by 24 hours of TGF-β1 stimulation and addition of empty fresh medium for the subsequent 24 h; D3 = fibroblasts after 18 days of culture followed by 24 hours of TGF-β1 stimulation and by treatment with vitamin D3 for the subsequent 24 h; EGCG = fibroblasts after 18 days of culture followed by 24 hours of TGF-β1 stimulation and by treatment with vitamin EGCG for the subsequent 24 h; D3 + EGCG = fibroblasts after 18 days of culture followed by 24 hours of TGF-β1 stimulation and by treatment with a combination of vitamin D3 and EGCG for the subsequent 24 h.
Fig. 1 shows the effect of vitamin D or EGCG alone or combined after 24 hours of incubation on the production of fibronectin in fibroblasts cells in vitro: Vitamin D alone slightly increases fibronectin production. EGCG alone reduces the production of fibronectin. Production of fibronectin is significantly reduced by vitamin D in combination with EGCG.
Fig. 2 shows the effect of vitamin D or EGCG alone or combined after 24 hours of incubation on the production of collagen in fibroblasts cells in vitro: Vitamin D alone reduces the production of collagen. EGCG alone reduces the production of collagen. Production of collagen is significantly reduced by vitamin D in combination with EGCG. The increased reduction of collagen is given by the combination of vitamin D plus EGCG.

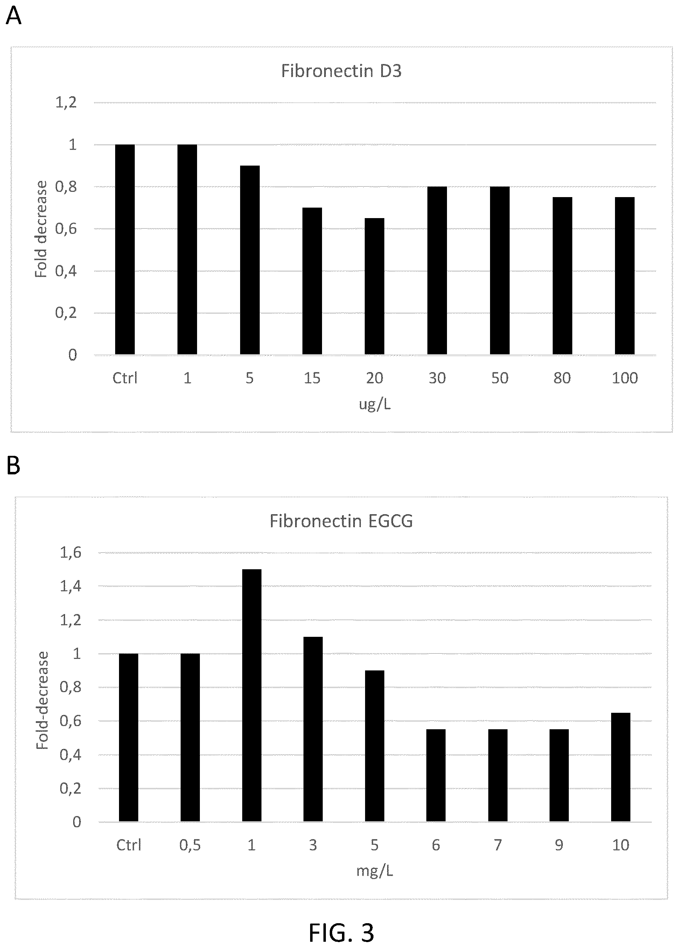

Fig. 3 shows fibronectin release in cultured fibroblasts in presence of Vitamin D3 (A) or EGCG (B) at different concentrations after 24 hours. Ctrl = fibroblasts after 18 days of culture followed by 24 hours of TGF-β1 stimulation and addition of empty fresh medium for the subsequent 24 h.

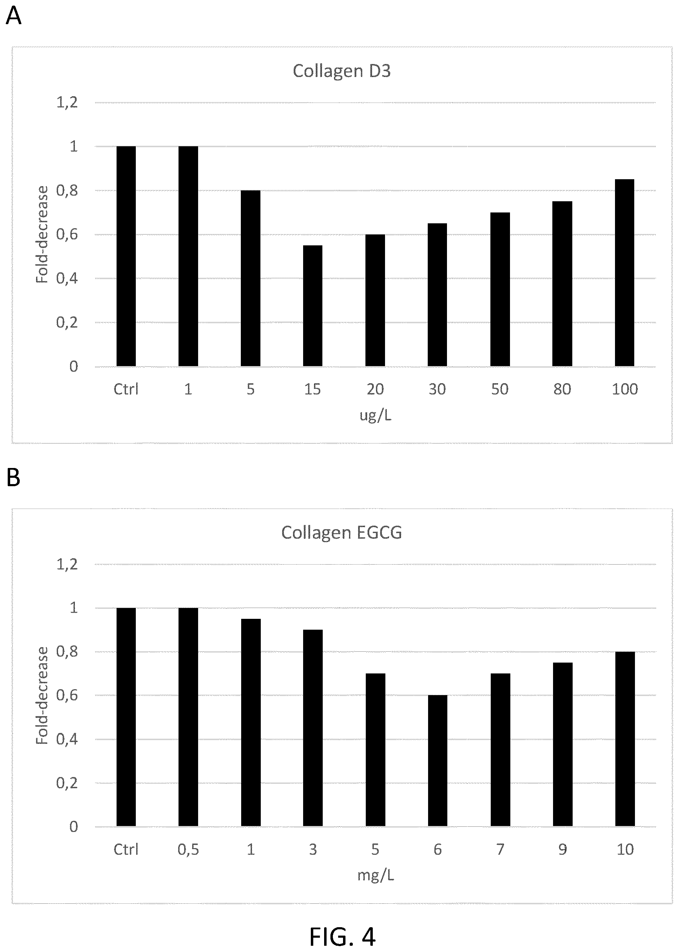

Fig. 4 shows collagen release in cultured fibroblasts in presence of Vitamin D3 (A) or EGCG (B) at different concentrations after 24 hours. Ctrl = fibroblasts after 18 days of culture followed by 24 hours of TGF-β1 stimulation and addition of empty fresh medium for the subsequent 24 h.

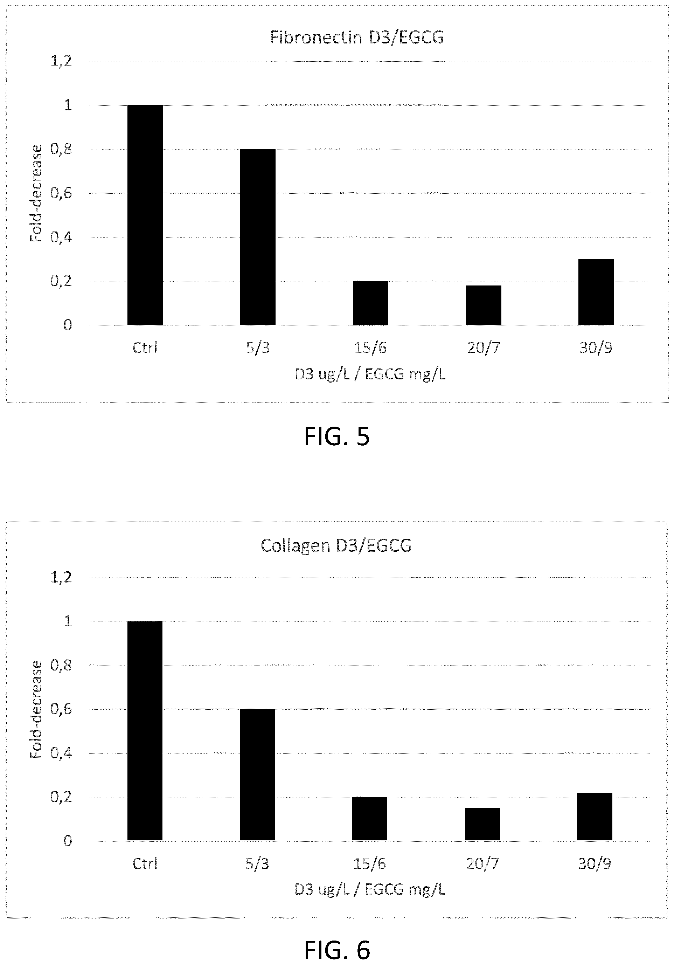

Fig. 5 and 6 respectively show fibronectin and collagen release in cultured fibroblasts in presence of the combination of Vitamin D3 and EGCG after 24 hours. Ctrl = fibroblasts after 18 days of culture followed by 24 hours of TGF-β1 stimulation and addition of empty fresh medium for the subsequent 24 h.
In the x-axys labels, the first number (preceding the slash) refers to the concentration of Vitamin D3 in ug/L and the second number (following the slash) refers to the concentration of EGCG in mg/L, e.g. 5/3 indicates that 5 ug/L of Vitamin D3 and 3 mg/L of EGCG are administered.

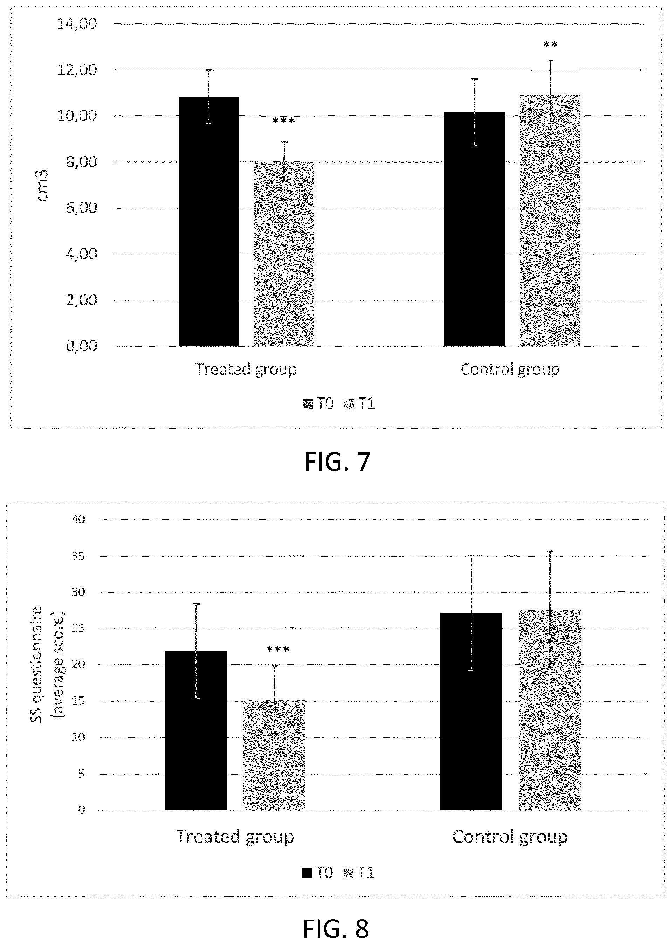

Fig. 7 shows volume of myomas (i.e. uterine fibroids) in the treated and control patient groups at baseline (T₀) and after the 4-month study period (T₁). Volume of myomas is indicated (mean ± SEM) as cm³. Treated group = vitamin D + EGCG for 4 months; control group = no treatment for 4 months. A p-value ≤0.05 was considered statistically significant. *Significance T₁ vs T₀. Statistical test = unpaired t-test.
Fig. 8 shows the average score of the severity of symptoms (SS) in women with myomas. Treated group (vitamin D + EGCG for 4 months); control group (no treatment for 4 months); T₀ = baseline; T₁ = after the 4-month study period. A p-value ≤0.05 was considered statistically significant. *Significance T₁ vs T₀. Statistical test = unpaired t-test.

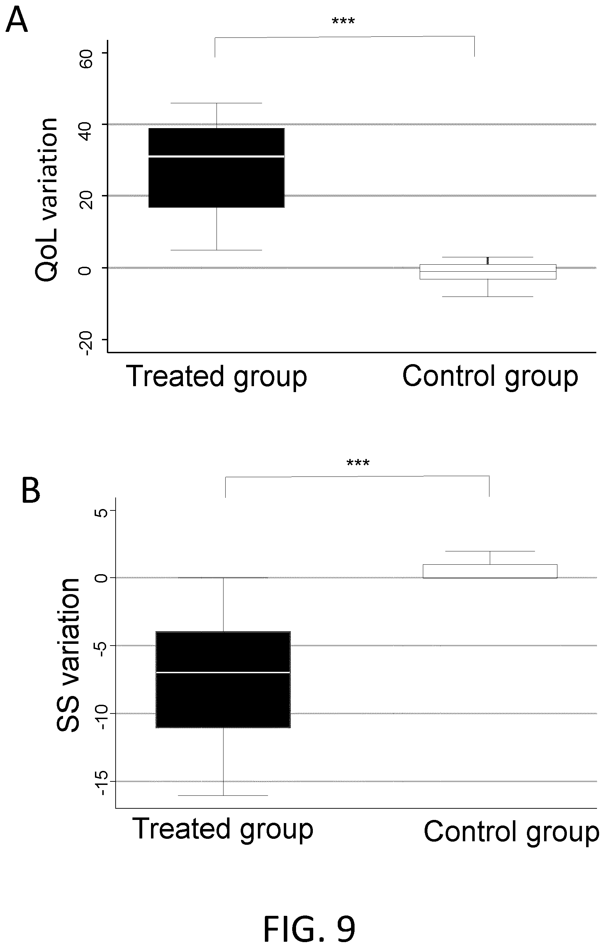

Fig. 9 shows variation of quality of life (QoL) and severity of symptoms (SS) in women with myomas. Data are presented as box plots. A: Variation of quality of life (QoL). B: Variation of severity of symptoms (SS). Variation is determined between the changes from T₀ to T₁ in each group (i.e. as the difference of the score at T₁ - the score at T₀), through the evaluation of the patients' responses in the questionnaires. Treated group (vitamin D + EGCG for 4 months); control group (no treatment for 4 months). Wilcoxon-Mann-Whitney test were used for the analyses. A p-value ≤0.05 was considered statistically significant.

### Detailed description of the invention

The present invention is defined by the claims. Any subject-matter falling outside the scope of the claims is provided for information only. Methods of treatment of the human body by therapy, referred to in this description, are not part of the present invention as such, but are described here in relation to compounds and pharmaceutical compositions for use in said methods for treatment of the human body by therapy, according to the present invention.

The following provides definitions of various terms and expressions used in the present application to describe the invention. It is to be understood that definitions of terms and expressions used in more than one aspect or embodiment of the invention apply equally to any aspect or embodiment of the invention described herein in which those terms and expressions appear. This applies equally regardless of where, i.e. which section, within the present application such terms are defined or discussed.

In this application, the use of the singular (e.g. "a" or "the") may include the plural unless specifically stated otherwise. As used herein, "one or more" is a synonym of "at least one" and is to be intended as referring to one agent or to any combination of more than one agent. As used hereinbelow, the singular "an agent" or "the agent" is intended to refer to the one or more agent as defined above. Where the ingredients are administered with an additional agent, different from the one or more agent, this additional agent is defined as "further agent". Further, the use of the term "including" as well as other grammatical forms such as "includes" and "included", is not limiting. As used herein the term "comprising" has the broad standard meaning "including", "encompassing", or "containing". It includes the explicitly recited element(s), and also allows, but does not require, the presence of other or another element(s) not recited. In addition to this broad meaning, as used herein, the term "comprising" also encompasses the limiting meaning "consisting of", according to which only the explicitly recited element(s), and no other(s) are present. In addition, the term "comprising" also includes the meaning of "consisting essentially of", which means that other element(s) may be present beyond those explicitly recited, provided the additionally present element(s) does not alter the technical effect achieved by the explicitly recited element(s).

As used herein the terms "about", "approximately" and synonyms thereof when referring to a particular value, e.g. an endpoint or endpoints of a range, encompass and disclose, in addition to the specifically recited value itself, a certain variation around the specifically recited value. Such a variation may for example arise from normal measurement variability, e.g. in the weighing or apportioning of various substances by methods known to the skilled person. The terms "about", "approximately" and synonyms thereof shall be understood as encompassing and disclosing a range of variability above and below an indicated specific value, said percentage values being relative to the specific recited value itself, as follows. The term "about" may encompass and disclose variability of ± 5.0%, ± 4.9%, ± 4.8%, ± 4.7%, ± 4.6%, ± 4.5%, ± 4.4%, ± 4.3%, ± 4.2%, ± 4.1%, ± 4.0%, ± 3.9%, ± 3.8%, ± 3.7%. ± 3.6%, ± 3.5%, ± 3.4%, ± 3.3%, ± 3.2%, ± 3.1%, ± 3.0%, ± 2.9%, ± 2.8 %, ± 2.7%, ± 2.6%, ± 2.5%, ± 2.4%, ± 2.3%, ± 2.2%, ± 2.1%, ± 2.0%, ± 1.9%, ± 1.8 %, ± 1.7%, ± 1.6%, ± 1.5%, ± 1.4%, ± 1.3%, ± 1.2%, ± 1.1%, ± 1.0%, ± 0.9%, ± 0.8 %, ± 0.7%, ± 0.6%, ± 0.5%, ± 0.4%, ± 0.3%, ± 0.2%, ± 0.1. The terms "about", "approximately" and synonyms thereof, in reference to the particular recited value, may encompass and disclose that exact particular value itself, irrespective of any explicit mention that this exact particular value is included; even in the absence of an explicit indication that such terms include the particular exact recited value, this exact particular value is still included in the range of variation created by said terms, and is therefore disclosed.

As is set out above, the present invention relates to Vitamin D and an agent such as EGCG as defined above for use in a method of controlling/preventing/treating fibroids in a subject. In a related aspect, the present invention pertains to a method of controlling/preventing/treating fibroid in a subject in need or potential need thereof, comprising the step of administering Vitamin D and an agent such as EGCG as defined above to said subject. In a further related aspect, the present invention pertains to a use of Vitamin D and an agent such as EGCG as defined above in the manufacture of a medicament for treating/preventing/controlling fibroids in a subject.

As used herein, the term "subject" refers to a mammal, preferably a human. The subject may be in need, potential need or suspected need of preventing/treating/controlling a fibroid.

The subject may be in need of treating a fibroid. In such cases, this need will typically have been previously determined by obtaining a diagnosis of fibroid. In such cases, in which a diagnosis or diagnoses of a pathological condition or pathological conditions already exists, the invention as set out herein may be implemented to treat existing pathology (e.g. uterine fibroid), while simultaneously preventing the development of at least one symptom thereof (e.g. excessive menstrual bleeding, dysmenorrhea, chronic pelvic pain, and pressure symptoms such as a sensation of bloatedness, and bowel disturbance). It is however also contemplated that the subject matter of the invention as set out herein can be applied to subjects suspected of having a need thereof. Such subjects may be at risk for developing this condition. For instance, overweight or obese subjects, subjects with a positive diagnosis of endometriosis, or high blood pressure, and subjects with a family history of uterine fibroids or more generally black women that are more at risk for developing uterine fibroids.

Accordingly, as used herein, the term "treat" or grammatically related variants thereof such as "treatment", "treating" etc. means the amelioration, even temporarily, encompassing but not requiring the complete abolishment of a pathological state or of at least one symptom thereof. In the broadest sense, treatment of fibroid, by the medical uses and methods of treatment herein means achieving at least a partial reversion of dysfunctional fibrotic tissue to its previously functional state. In particular, treatment in the present sense encompasses restoration of proper organ architecture and function in organs in which fibrotic tissue has developed. For instance, in the case the organ is the uterus, treatment would encompass reduction of proliferation of fibroblast and extracellular matrix (ECM) protein in fibrotic tissue to restore functional uterine tissue.

As used herein, the term "fibroid" refers in particular to a condition in which noncancerous muscle tissue cells and fibrous connective tissue develop in a tissue. This fibroid can develop from an aberrant muscle cell in the tissue. A fibroid can be diagnosed during a routine examination or by any one or more of the following: X-ray, ultrasound or ultrasonography, magnetic resonance imaging, hysterosalpingography, hysteroscopy, biopsy, blood test. As used herein, "fibroid" is preferably a uterine fibroid. The majority of the volume of fibroids usually consists of fibronectin, collagen and/or proteoglycan. Therefore, the control, treatment and/or prevention of fibroids can be measured by the release of fibronectin, collagen and/or proteoglycan. In particular, keeping approximately constant, decreasing, inhibiting, and/or preventing the release of fibronectin, collagen and/or proteoglycan are indicative of fibroid control, treatment and/or prevention.

Such keeping approximately constant, decreasing, inhibiting, and/or preventing can be measured with respect to a proper control by techniques known in the art, including any known method of detecting and/or quantifying fibronectin, collagen and/or proteoglycan, such as Western Blot and enzyme immunoassay.

Proper controls include but are not limited to: healthy subjects, subjects having a fibroid and not being treated, subjects having a fibroid and being treated with a therapeutic approach different from that of the present invention, subjects having a fibroid and being treated with vitamin D only, subjects having a fibroid and being treated with an agent such as EGCG only.

As used herein, the terms "uterine fibroid", "uterine leiomyoma" and "myoma" are used interchangeably and refer to a fibroid as defined above located in the uterus. This fibroid tumor can develop from an aberrant muscle cell in the uterus, which multiplies rapidly because of the influence of estrogen of the uterus grows increasing the number of fibroblasts and ECM proteins (in particular fibronectin, collagen and/or proteoglycan), leading to an altered, pathological function. A uterine fibroid can be diagnosed during a routine examination or by any one or more of the following: X-ray, transvaginal or transabdominal ultrasound or ultrasonography, magnetic resonance imaging, hysterosalpingography, hysteroscopy, endometrial biopsy, blood test. In general, treatment of uterine fibroids may include: surgery, a gonadotropin-releasing hormone agonist (GnRHa), a selective progesterone receptor modulator (sPRMs), an anti-hormonal agent, a hormone, an anti-inflammatory painkiller and tranexamic acid, as defined above. Any of such treatments of uterine fibroids may be used in combination with vitamin D and the agent according to the present invention.

As used herein, the terms "prostate hyperplasia", "benign prostatic hyperplasia (BPH)", "prostatic enlargement" and "prostatic leiomyoma" refer to a fibroid as defined above located in the prostate. For example, "prostate hyperplasia" indicates an increase in the number of cells in the prostate and/or a noncancerous growth in size of the prostate gland. Prostatic hyperplasia may result in an increase in the deposition of extracellular matrix (ECM), i.e. in an increased release/level of fibronectin, collagen and/or proteoglycan. In other words, a prostatic hyperplasia is a prostate fibroid. The treatment of BPH is not encompassed by the claimed invention.

As used herein, the term "fibroadenoma" refers to a fibroid as defined above located in the breast. Preferably, it is a benign human breast tumor, characterized by proliferation of epithelial and fibroblast stromal components of the terminal ductal unit, i.e. in an increased release/level of fibronectin, collagen and/or proteoglycan. In other words, a fibroadenoma is a mammary fibroid.

As used herein, the term "prevent" or grammatically related variants thereof such as "prevention" refer to scenarios in which the medical uses and methods of treatment described herein are applied to avert the possible, suspected or expected occurrence of a fibroid. Such suspicion that a fibroid may derive (even in the absence of a corresponding diagnosis) from the subject history, for example if the subject had previously a fibroid. In such a scenario, there may exist a justified suspicion that a previously treated fibroid, may reoccur. Such suspicion that a fibroid may occur (even in the absence of a corresponding diagnosis) may apply also to subjects at risk of developing this condition, for instance, overweight or obese subjects, subjects with a positive diagnosis of endometriosis or with high blood pressure, and subjects with a family history of fibroids or more generally black women that are more at risk for developing uterine fibroids.

Vitamin D and the one or more agent or the composition as defined above may also be used to control the size and/or growth of the fibroid over a prolonged period of time, e.g. more than 1 month, approximately 4 months, more than 6 months, more than 1 year or more years. Such scenarios may apply for example, when myomas are asymptomatic or of smal size such that no interventions are prescribed, and patients are advised to control the size and growth of myomas, with routine specialist visits and ecografic analysis.

As used herein, the term "vitamin D" encompasses and discloses all substances commonly known as vitamin D, including the substances having chemical formulas C₂₇H₄₄O = Vitamin D3 (cholecalciferol), C₂₈H₄₄O = Vitamin D2 (ergocalciferol), C₂₇H₄₄O₂ = 25(OH)vitamin D (calcidiol), C₂₇H₄₄O₃ = 1,25 dihydroxy-vitamin D (calcitriol). Preferably, vitamin D refers to vitamin D3.

Sources of vitamin D include dietary intakes such as fish oils, fatty fish, fortified foods, and vitamin supplements.

As used herein, the term "vitamin B" encompasses and discloses all substances commonly known as vitamin B, including but not limited to: thiamin (vitamin B1), riboflavin, niacin, pantothenic acid, biotin, vitamin B6, folate, vitamin B12 (cyanocobalamin). Preferably, vitamin B refers to vitamin B6. Vitamin B6 in turn encompasses and discloses all substances commonly known as vitamin B6, such as Pyridoxine (PN), Pyridoxine 5'-phosphate (P5P), Pyridoxal (PL), Pyridoxal 5'-phosphate (PLP), Pyridoxamine (PM), Pyridoxamine 5'-phosphate (PMP), 4-Pyridoxic acid (PA), Pyritinol.

Sources of vitamin B6 include food of vegetable or animal origin, such as cereals, wholemeal flour, lentils, milk, avocado, dried fruit, peppers, spinach, broccoli, green beans, carrots, potatoes, bananas, sunflower seeds, celery seeds, offal, tuna, crustaceans, salmon, chestnut, eggs, soy, mushroom, chocolate, aromatic herbs, paprika, chili pepper, green-leafed vegetables, brewer's yeast, fish, peas.

As used herein, the term "EGCG" encompasses and discloses all substances commonly known EGCG, including the substance having chemical formula C₂₂H₁₈O₁₁, such as: epigallocatechin gallate (EGCG), epigallocatechin-3-gallate, (-)-epigallocatechin-3-gallate, (-)-gallocatechin-3-O-gallate (GCG), (-)-gallocatechin-3-gallate.

As used herein, the term "epicatechin gallate" (ECG) encompasses and discloses all substances commonly known as epicatechin gallate, including the substances having chemical formula: C₂₂H₁₈O₁₀, such as: (-)-epicatechin-3-gallate, (-)-catechin-3-gallate (CG) and (+)-catechin-3-gallate (CG).

As used herein, the term "catechin" encompasses and discloses all substances commonly known as catechin, including the substances having chemical formula C₁₅H₁₄O₆, such as: catechin, (+)-catechin (C), epicatechin, (-)-epicatechin (EC).

As used herein, the term "gallocatechin" encompasses and discloses all substances commonly known as gallocatechin, including the substances having chemical formula: C₁₅H₁₄O₇, such as: gallocatechin, (+)-gallocatechin (GC), epigallocatechin, (-)-epigallocatechin (EGC).

EGCG, ECG, catechin and gallocatechin are comprised in a variety of foods, any of which or any combination of which may be used in the present invention as a source of EGCG, ECG, catechin and/or gallocatechin. In particular, in the present invention, the source of EGCG, ECG, catechin and/or gallocatechin may be: green tea, white tea, oolong tea, black tea, carob flour, blackberries, apples, raspberries, pecan nuts, hazelnut, pistachio, blueberries, plums, peaches, avocados, grapes, strawberries, cocoa, chocolate, wine, fruit, vegetables and/or onions. Carob flour may be obtained from the fruit of the Ceratonia plant.

Preferably, the source of EGCG, ECG, catechin and/or gallocatechin is tea, in particular: green tea, white tea, oolong tea and black tea. Any tea plant may be used as a source of EGCG, ECG, catechin and/or gallocatechin in the present invention.

Preferably, Camellia sinensis is used. Any part of a tea plant or the entire plant may be used as a source of EGCG, ECG, catechin and/or gallocatechin in the present invention. Preferably, leaves of a tea plant are used. Preferably, leaves of Camellia sinensis are used as a source of EGCG, ECG, catechin and/or gallocatechin in the present invention.

Tea leaves may comprise different amounts of EGCG, ECG, catechin and/or gallocatechin in particular from 30 wt% to 95 wt%, for example 30 wt%, 45 wt%, 50 wt% and 95 wt%. Such amounts can be determined by titration and depend on the purity and processing of the raw material.

Preferably, EGCG, ECG, catechin and/or gallocatechin is used in the present invention as a tea dry extract, preferably a green tea dry extract.

In an embodiment the present invention refers to a combination of Vitamin D and EGCG.

In a preferred embodiment the present invention refers to a combination of Vitamin D, EGCG and vitamin B6.

In one embodiment, the present invention refers to the respective concentration: Composition of 1 tablet

| | |
|---|---|
| Extract of green tea: | 333.35 mg (Tit. 45% in Epigallocatechine gallate (EGCG) 150 mg) |
| Vitamin B6 | 5 mg |
| Vitamin D | 25 µg |

In one embodiment, the present invention refers to a daily intake of:

| | |
|---|---|
| Extract of green tea: | 666.70 mg (Tit. 45% in Epigallocatechine gallate (EGCG) 300 mg) |
| Vitamin B6 | 10 mg |
| Vitamin D | 50 µg corresponding to 2.000 IU |

The substances of the present invention, in particular vitamin D, vitamin B, EGCG, ECG, catechin and gallocatechin may exist and be used according to the invention in any salt, solvate, stereoisomeric, zwitterionic, tautomeric and/or isotopic form.

Salts of the substances of the present invention are preferably pharmaceutically acceptable. Suitable pharmaceutically acceptable salts comprise conventional nontoxic salts obtained by salification of an agent of the present invention with inorganic acids (e.g. hydrochloric, hydrobromic, sulphuric, or phosphoric acids), or with organic acids (e.g. acetic, propionic, succinic, benzoic, sulfanilic, 2-acetoxy-benzoic, cinnamic, mandelic, salicylic, glycolic, lactic, oxalic, malic, maleic, malonic, fumaric, tartaric, citric, p-toluenesulfonic, methanesulfonic, ethanesulfonic, or naphthalensulfonic acids). For reviews on suitable pharmaceutical salts see [Berge S. M. et al., J. Pharm. Sci. 1977, 66, 1-19; Gould P. L. Int. J. Pharm 1986, 33, 201-217; Bighley et al. Encyclopedia of Pharmaceutical Technology, Marcel Dekker Inc, New York 1996, Volume 13, page 453-497; and Remington "The Science and Practice of Pharmacy", Lippincott Williams & Wilkins, 2000].

In addition, pharmaceutically acceptable base addition salts can be formed with a suitable inorganic or organic base such as triethylamine, ethanolamine, triethanolamine, dicyclohexylamine, ammonium hydroxide, pyridine. The term "inorganic base," as used herein, has its ordinary meaning as understood by one of ordinary skill in the art and broadly refers to an inorganic compound that can act as a proton acceptor. The term "organic base," as used herein, also has its ordinary meaning as understood by one of ordinary skill in the art and broadly refers to an organic compound that can act as a proton acceptor.

Other suitable pharmaceutically acceptable salts include pharmaceutically acceptable alkali-metal or alkaline-earth-metal salts such as sodium, potassium, calcium or magnesium salts.

The invention refers to the use of all possible stoichiometric and nonstoichiometric forms of the salts of the substances of the present invention.

In addition, the substances of the present invention may exist in nonsolvated as well as in solvated forms with pharmaceutically acceptable solvents such as water, EtOH and the like.

Certain substances of the present invention, in particular EGCG, ECG, catechin and gallocatechin, may exist in stereoisomeric forms (e.g. they may contain one or more asymmetric carbon atoms). All stereoisomers of the substances of the present invention, in particular EGCG, ECG, catechin and gallocatechin, may be used in the treatment of fibroids according to the present invention. The use of individual stereoisomers (enantiomers and diastereomers) and mixtures of these are included within the scope of the present invention. Racemic mixtures may be separated to give their individual enantiomer using preparative HPLC using a column with chiral stationary phase or resolved to yield individual enantiomers utilizing methods known to those skilled in the art.

The substances of the present invention may exist in zwitterionic form, all of which may be used in the treatment of fibroids according to the present invention. Likewise, it is understood that substances of the present invention may exist in tautomeric forms and their use is also included within the scope of the present invention.

The invention also includes the use of all suitable isotopic variations of substances of the present invention. An "isotopic variation" of a substance of the invention is defined as one in which at least one atom is replaced by an atom having the same atomic number but an atomic mass different from the atomic mass usually found in nature. Examples of isotopes that can be incorporated into substances of the invention include isotopes such as ²H, ³H, ¹³C, ¹⁴C, ¹⁵N, ¹⁷O, ¹⁸O, ³¹P, ³²P, ³⁵S, ¹⁸F and ³⁶Cl. Certain isotopic variations of the invention, for example, those in which a radioactive isotope such as ³H or ¹⁴C is incorporated, are useful in drug and/or substrate tissue distribution studies. Further, substitution with isotopes such as deuterium ²H, may afford certain therapeutic advantages resulting from greater metabolic stability. Isotopic variations of the substances of the invention can generally be prepared by conventional procedures such as by the illustrative methods or by the preparations described in the examples hereafter using appropriate isotopic variations of suitable reagents.

As used herein, the term "combination" usually refers to the coupling of the agent as defined above and at least vitamin D for the purpose of their co-administration to a subject within a given treatment regimen. A given treatment regimen may comprise multiple, repeated co-administrations of Vitamin D and the agent as defined above over a predetermined time course, e.g. over 1 month, over 2 months, over 3 months, over 4 months, over 5 months or over 6 months or more, a time course of at least 4 months or of approximately 4 months being preferred. The co-administration of the agent as defined above and at least vitamin D may be repeated multiple times daily, for example once, twice, 3 times, 4 times, 5 times or more, a repetition about twice daily being preferred. That is, the co-administration of Vitamin D and the agent as defined above is repeated over a predetermined length of time, and the sum of the instances of co-administration over this predetermined length of time constitutes the prophylactic or therapeutic regimen.

The coupling of Vitamin D and the agent, for instance as defined above meant by a "combination" of these two substances, need not be physical, nor need it imply simultaneity of administration. Administration of Vitamin D and the agent or of a combination thereof as defined above therefore encompasses and discloses multiple possibilities regarding the route and timing of administration of the respective substances. Encompassed and disclosed is for example the coupling of Vitamin D and the agent as defined above for simultaneous administration by the same route, simultaneous administration by different routes, chronologically staggered (i.e. non-simultaneous) administration by the same route, or chronologically staggered (i.e. non-simultaneous) administration by different routes. Any of the administration routes described herein may be combined in any way, although it will generally be preferable that the route will be oral. Especially preferable is simultaneous administration of Vitamin D and the agent as defined above by the oral route, e.g. when Vitamin D and the agent as defined above are present in a composition, e.g. in one of the inventive compositions set out herein, e.g. in the form of a tablet, hard capsule, soft gel capsule, or powder, e.g. in the form of a sachet, for ingestion.

Simultaneous administration of Vitamin D and the agent as defined above by the same route might for example take the form of Vitamin D and the agent as defined above being comprised in the same physical composition, that composition being administered to, e.g. ingested by, the subject so that the Vitamin D and the agent as defined above enter the body by the same route, e.g. by the oral route, at the same time. Coupling of Vitamin D and the agent as defined above for staggered (i.e. non-simultaneous) administration within a given co-administration is however also possible and is also encompassed within the present invention and disclosed in the term "combination". The order of administration of Vitamin D and the agent as defined above is not particularly important; the chronologically staggered (i.e. non-simultaneous) administration of Vitamin D and the agent as defined above might encompass prior administration of the agent as defined above and subsequent administration of Vitamin D, or prior administration of Vitamin D and subsequent administration of the agent as defined above. For example, staggered (i.e. non-simultaneous) administration of Vitamin D and the agent as defined above by the same route may take the form of initial oral administration of Vitamin D, followed by oral administration of the agent as defined above; coupling of Vitamin D and the agent as defined above in this way falls within the scope of the present invention and within the meaning of, and is disclosed by, "combination" as used herein. Conversely, staggered (i.e. non-simultaneous) administration of Vitamin D and the agent as defined above by different routes might for instance take the form of initial oral administration of Vitamin D, followed by administration of the agent as defined above by e.g. suppository; coupling of Vitamin D and the agent as defined above in such ways also falls within the scope of the present invention and within the meaning of, and is disclosed by, "combination" as used herein.

As used herein, the term "non-simultaneously" means that Vitamin D and the agent as defined above, are administered in a chronologically staggered manner, i.e. at different times. The administration refers to a respective co-administration of Vitamin D and the agent as defined above within a broader regimen of treatment or prophylaxis. For instance, one embodiment entails administering the agent as defined above to the subject prior to administering Vitamin D to the subject. Another embodiment entails administering the agent as defined above to the subject following administering Vitamin D to the subject. It is understood that such non-simultaneous administration denotes any respective instance of coadministration of Vitamin D and the agent as defined above within the broader context of the overall treatment regimen. As mentioned herein, regardless of the order of administration, that is e.g. the agent as defined above first and vitamin D, second, or *vice versa,* the respective combined administration may be by the same or different routes.

In a further embodiment Vitamin D and the agent as defined above are administered to the subject simultaneously. As mentioned above, the simultaneous administration of Vitamin D and the agent as defined above can be effected by the same or different routes. Most typically, it will be most advantageous and convenient to effect the simultaneous administration of Vitamin D and the agent as defined above by the same route, preferably by an oral administration route. This will most often be accomplished by combining Vitamin D and the agent as defined above to be administered in the same composition, for example in the form of a tablet, including but not limited to an effervescent tablet, a powder (especially presented in the form of a sachet), a hard capsule, a soft gel capsule, a syrup, a cachet, a troche, or a lozenge.

There are no particular restrictions on the duration of time between administration of Vitamin D and the agent as defined above in the event the co-administration is chronologically staggered, i.e. non-simultaneous, however it will generally be most effective and convenient when any interim between administering Vitamin D and the agent as defined above is brief, approaching simultaneity, e.g. on the order of minutes. In some cases such an interim may extend up to about an hour or several hours between respective administrations. In some cases, then, in the event that the coadministration of Vitamin D and the agent as defined above is chronologically staggered, the interim between administration in some instances may be as long as 12 hours. However, even in the event Vitamin D and the agent as defined above are administered in a chronologically staggered manner, whether by the same or different routes, the term "combination" means that a given co-administration of both Vitamin D and the agent as defined above within any regimen will have been completed, i.e. both substances will have been administered, by the time the respective next coadministration of Vitamin D and the agent as defined above (which itself may again be either simultaneous or chronologically staggered by the same or different routes in a manner corresponding to are different from the chronological staggering and routes of the previous co-administration) within the same regimen ensues.

As used herein, the term "composition" encompasses and discloses any physical entity comprising or consisting of or consisting essentially of the respective recited substances, e.g. comprising or consisting of or consisting essentially of the Vitamin D and the agent as defined above. The physical form of the composition is not restricted. For example, the term "composition" encompasses and discloses a powder in which the recited substances are each present in powder form. As a further example, the term "composition" also encompasses and discloses a liquid solution in which the recited substances are present in solubilized form. As a further example, the term "composition" also encompasses and discloses an emulsion in which the recited substances are present. As a further example, the term "composition" also encompasses and discloses a suspension in which the recited substances are present. As a further example, the term "composition" also encompasses and discloses mixtures in which the agent as defined above is in one form, e.g. a solid such as a powder, while the Vitamin D is in another form, e.g. a liquid. In particular, the term "composition" may be a "pharmaceutical composition" as defined herein below, and may be formulated for a desired route of administration. As used and disclosed herein, the term "composition" may also be a composition suitable for oral delivery, e.g. in the form of a tablet, including but not limited to an effervescent tablet or a multilayer tablet, a powder, e.g. in the form of a sachet, a hard capsule, a soft gel capsule, a syrup, a cachet, a troche, or a lozenge, a pastille, e.g. a gummy pastille, a salve, or a liquid preparation. In certain especially preferred embodiments of the invention, the "composition" comprising Vitamin D and the agent as defined above may be in the form of a soft gel capsule. In certain other especially preferred embodiments of the invention, the "composition" comprising Vitamin D and the agent as defined above maybe in the form of a powder. The term "composition" may also be a composition suitable for delivery by a non-oral route, for example in the form of a suppository, a tablet, a hard capsule, a soft gel capsule, a cream, a gel, a patch or a liquid. Further dosage forms of the composition as well as referred routes of administration are set out herein below. In the medical uses and treatment methods described herein, it is contemplated that Vitamin D and the agent as defined above may be administered in combination with one or more additional substances.

For example, Vitamin D and the agent as defined above may be administered in combination with a source of Vitamin B6. The inclusion of a source of Vitamin B6 for administration in combination with Vitamin D and the agent as defined above according to the medical uses and methods of treatment set out herein may be especially advantageous in case of fibroids. Vitamin B6 intake contributes to the regulation of hormonal activity which is considered a risk factor for uterine fibroids. One such source of Vitamin B6 refers to a group of chemically similar compounds: Pyridoxine (PN), Pyridoxine 5'-phosphate (P5P), Pyridoxal (PL), Pyridoxal 5'-phosphate (PLP), Pyridoxamine (PM), Pyridoxamine 5'-phosphate (PMP), 4-Pyridoxic acid (PA), Pyritinol.

For instance, one embodiment of the medical uses and methods of treatment described herein may entail combined administration of about 150 mg EGCG and about 25 µg Vitamin D. A further embodiment of the medical uses and methods of treatment described herein may entail combined administration of about 150 mg EGCG, about 25 µg Vitamin D and 5 mg Vitamin B6.

As another example, Vitamin D and the agent as defined above in the medical uses and methods of treatment described herein may also be administered in combination with one or more polyphenols. The inclusion of one or more polyphenols for administration in combination with Vitamin D and the agent as defined above according to the medical uses and methods of treatment set out herein may be especially advantageous in case of fibroids. As example, polyphenols can derive from cocoa, olive oil, grape and pomegranate. Polyphenols are known to have a positive effect on fibroids; olive oil polyphenols, such as hydroxytyrosol, are known for potent antioxidant and anti-inflammatory effects.

Accordingly, in one embodiment, the inventive composition is a pharmaceutical composition. The compositions of the invention, including pharmaceutical compositions, may further comprise at least one pharmaceutically acceptable ingredient. While the inventive composition, including the pharmaceutical composition, may itself be administered to a subject, it will be understood that adding one or more pharmaceutically acceptable ingredients beyond Vitamin D and the agent as defined above may be advantageous in rendering the composition more suitable for direct administration to a subject by a given predetermined route. Accordingly, the "pharmaceutical composition", may be formulated to comprise, in addition to Vitamin D and the agent as defined above, a pharmaceutically acceptable ingredient which renders the composition more suitable or especially suitable for direct administration to a subject by a given route, without further workup. This suitability may pertain to a number of different administration routes, including oral, parenteral, transmucosal, vaginal or perivaginal, topical, transdermal or intravesical, as further set out in the following. Where present, and by way of non-limiting illustration, such ingredients as well as their advantageous impact on the suitability of the inventive composition, including pharmaceutical composition, supplement, food product and drink product, for different routes of administration are set out below.

### a) Formulation suitable for oral administration

A composition suitable for oral administration may be prepared, packaged, or sold in the form of a discrete solid dose unit including a sachet (e.g. a powder in a sachet), a tablet, a hard or soft capsule, a cachet, a troche, or a lozenge, each containing a predetermined amount Vitamin D and the agent as specified herein. Other formulations suitable for oral administration include a powdered or granular formulation, an aqueous or oily suspension, an aqueous or oily solution, or an emulsion. As used herein, an "oily" liquid comprises a carbon-containing liquid molecule that exhibits a less polar character than water.

It is especially preferred that the discrete solid dose unit of the inventive composition is in the form of a powder, especially presented in the form of a sachet. As used herein, the term "sachet" refers to a sealed pouch containing the inventive composition. The pouch may be made of paper, wax paper, plasticized paper, or a combination of paper and foil. The material out of which the sachet is fashioned is preferably impervious to ambient moisture and other potential atmospheric contaminants so that, by sealing said sachet, the inventive composition in the form of a powder contained therein remains in a free flowing form until use.

A tablet comprising Vitamin D and the agent as defined above in any of the above concentrations, may for example be made by compressing or molding Vitamin D and the agent as defined above with one or more additional ingredients. Compressed tablets may be prepared by compressing, in a suitable device, Vitamin D and the agent as defined above in a free flowing form such as a powder or granular preparation, optionally mixed with one or more of a binder, a lubricant, an excipient, a surface active agent, and a dispersing agent. Molded tablets may for example be made by molding, in a suitable device, Vitamin D and the agent as defined above, a carrier, and at least sufficient liquid to moisten the mixture.

Acceptable excipients used in the manufacture of tablets include, but are not limited to, inert diluents, granulating and disintegrating agents, binding agents, and lubricating agents. Known dispersing agents include, but are not limited to, potato starch and sodium starch glycolate. Known surface active agents include, but are not limited to, sodium lauryl sulfate. Known diluents include, but are not limited to, calcium carbonate, sodium carbonate, lactose, microcrystalline cellulose, calcium phosphate, calcium hydrogen phosphate, and sodium phosphate. Known granulating and disintegrating agents include, but are not limited to, corn starch and alginic acid. Known binding agents include, but are not limited to, gelatin, acacia, pre-gelatinized maize starch, polyvinylpyrrolidone, and hydroxypropyl methylcellulose. Known lubricating agents include, but are not limited to, magnesium stearate, stearic acid, silica, and talc.

Tablets may be non-coated or they may be coated using known methods to achieve delayed disintegration in the gastrointestinal tract of a subject, thereby providing sustained release and absorption of Vitamin D and the agent as defined above. By way of example, a material such as glyceryl monostearate or glyceryl distearate may be used to coat tablets. Tablets may further comprise a sweetening agent, a flavoring agent, a coloring agent, a preservative, or some combination of these in order to provide attractive and palatable preparation.

Hard capsules comprising Vitamin D and the agent as defined above may be made using a physiologically degradable composition, such as gelatin or cellulose derivatives. Such hard capsules comprise Vitamin D and the agent as defined above, and may further comprise additional ingredients including, for example, an inert solid diluent such as calcium carbonate, calcium phosphate, or kaolin.

Soft gelatin capsules comprising Vitamin D and the agent as defined above may be made using a physiologically degradable composition, such as gelatin combined with a plasticizer (i.e. glycerol) as basic component of the soft gelatin shell. Soft gelatin capsules may contain a liquid or semisolid solution, suspension, or microemulsion preconcentrate. The soft capsule filling comprises Vitamin D and the agent as defined above, which may be mixed with water or an oil medium such as peanut oil, liquid paraffin, olive oil, soybean oil, sunflower oil, a lecithin such as for example soy lecithin or sunflower lecithin, medium chain triglycerides, polyglycerol oleate, beeswax, mono- and diglycerides of fatty acids, or combinations of any of the above.

Liquid formulations that are especially suitable for oral administration may be prepared, packaged, and sold either in liquid form or in the form of a dry product intended for reconstitution with water or another suitable vehicle prior to ingestion.

Liquid suspensions may be prepared using conventional methods to achieve suspension of the active ingredient(s) in an aqueous or oily vehicle. Aqueous vehicles include, for example, water and isotonic saline. Oily vehicles include, for example, almond oil, oily esters, ethyl alcohol, vegetable oils such as arachis, olive, sesame, or coconut oil, fractionated vegetable oils, and mineral oils such as liquid paraffin. Liquid suspensions may further comprise one or more additional ingredients including, but not limited to, suspending agents, dispersing or wetting agents, emulsifying agents, demulcents, preservatives, buffers, salts, flavorings, coloring agents, and sweetening agents. Oily suspensions may further comprise a thickening agent. Known suspending agents include, but are not limited to, sorbitol syrup, hydrogenated edible fats, sodium alginate, polyvinylpyrrolidone, gum tragacanth, gum acacia, and cellulose derivatives such as sodium carboxymethylcellulose, methylcellulose, hydroxypropyl methylcellulose. Known dispersing or wetting agents include, but are not limited to, naturally occurring phosphatides such as lecithin, condensation products of an alkylene oxide with a fatty acid, with a long chain aliphatic alcohol, with a partial ester derived from a fatty acid and a hexitol, or with a partial ester derived from a fatty acid and a hexitol anhydride (e.g., polyoxyethylene stearate, heptadecaethyleneoxycetanol, polyoxyethylene sorbitol monooleate, and polyoxyethylene sorbitan monooleate, respectively). Known emulsifying agents include, but are not limited to, lecithin and acacia. Known preservatives include, but are not limited to, methyl, ethyl, or n-propyl para-hydroxybenzoates, ascorbic acid, and sorbic acid. Known sweetening agents include, for example, glycerol, propylene glycol, sorbitol, sucrose, and saccharin. Known thickening agents for oily suspensions include, for example, beeswax, hard paraffin, and cetyl alcohol.

Powdered and granular formulations of a composition suitable for application in the present invention, e.g. a pharmaceutical composition, may be prepared using known methods. Such formulations may be administered directly to a subject, used, for example, to form sachet or tablets, to fill capsules, or to prepare an aqueous or oily suspension or solution by addition of an aqueous or oily vehicle thereto. Each of these formulations may further comprise one or more of dispersing or wetting agent, a suspending agent, and a preservative. Additional excipients, such as fillers and sweetening, flavoring, or coloring agents, may also be included in these formulations. A composition may also be prepared, packaged, or sold in the form of oil-in-water emulsion or a water-in-oil emulsion. The oily phase may be a vegetable oil such as olive or arachis oil, a mineral oil such as liquid paraffin, or a combination of these. Such compositions may further comprise one or more emulsifying agents such as naturally occurring gums such as gum acacia or gum tragacanth, naturally occurring phosphatides such as soybean or lecithin phosphatide, esters or partial esters derived from combinations of fatty acids and hexitol anhydrides such as sorbitan monooleate, and condensation products of such partial esters with ethylene oxide such as polyoxyethylene sorbitan monooleate. These emulsions may also contain additional ingredients including, for example, sweetening or flavoring agents.

Methods for impregnating or coating a material with a chemical composition are known in the art, and include, but are not limited to methods of depositing or binding a chemical composition onto a surface, methods of incorporating a chemical composition into the structure of a material during the synthesis of the material (e.g. such as with a physiologically degradable material), and methods of absorbing an aqueous or oily solution or suspension into an absorbent material, with or without subsequent drying.

### b) Formulation suitable for parenteral administration

For parenteral administration, a composition comprising Vitamin D and the agent as defined above may be formulated for injection or infusion, for example, intravenous, intramuscular or subcutaneous injection or infusion, or for administration in a bolus dose and/or continuous infusion. Suspensions, solutions or emulsions in an oily or aqueous vehicle, optionally containing other agents such as suspending, stabilizing and/or dispersing agents such as those mentioned above, may be used.

A composition comprising Vitamin D and the agent as defined above may be rendered especially suitable for parenteral administration by formulation with an acceptable carrier, such as sterile water or sterile isotonic saline. Such formulations may be prepared, packaged, or sold in a form suitable for bolus administration or for continuous administration. Injectable formulations may be prepared, packaged, or sold in unit dosage form, such as in ampoules, crushable or otherwise, or in multi-dose containers containing a preservative. Compositions especially suitable for parenteral administration include, but are not limited to, suspensions, solutions, emulsions in oily or aqueous vehicles, pastes, and implantable sustained-release or biodegradable formulations. Such compositions may further comprise one or more additional ingredients including, but not limited to, suspending, stabilizing, or dispersing agents. In one embodiment of a composition which is especially suitable for parenteral administration, the active ingredient is provided in dry (e.g. powder or granular) form for reconstitution with a suitable vehicle (e.g. sterile pyrogen free water) prior to parenteral administration of the reconstituted composition.

A composition suitable for application in the present invention may be prepared, packaged, or sold in the form of a sterile injectable aqueous or oily suspension or solution. This suspension or solution may be formulated according to the known art, and may comprise, in addition to the active ingredient(s), additional ingredients such as the dispersing agents, wetting agents, or suspending agents described herein. Such sterile injectable compositions may be prepared using a non toxic, parenterally acceptable diluent or solvent, such as water or 1,3-butanediol, for example. Other acceptable diluents and solvents include, but are not limited to, Ringer's solution, isotonic sodium chloride solution, and fixed oils such as synthetic mono- or diglycerides. Other usual parentally-administrable formulations include those that comprise the active ingredient in microcrystalline form, in a liposomal preparation, or as a component of a biodegradable polymer system. Compositions for sustained release or implantation may comprise acceptable polymeric or hydrophobic materials such as an emulsion, an ion exchange resin, a sparingly soluble polymer, or a sparingly soluble salt.

### c) Formulation suitable for transmucosal administration

A composition comprising Vitamin D and the agent as defined above may be formulated to be suitable for transmucosal administration. The formulation may include any substances or dosage unit suitable for application to mucosal tissue. For example, the selected active agent may be administered to the buccal mucosa in an adhesive tablet or patch, sublingually administered by placing a solid dosage form under the tongue, lingually administered by placing a solid dosage form on the tongue, administered nasally as droplets or a nasal spray, a non-aerosol liquid formulation, or a dry powder, placed within or near the rectum ("transrectal" formulations), or administered to the urethra as a suppository, ointment, or the like.

### d) Formulation suitable for vaginal or perivaginal administration

A composition comprising Vitamin D and the agent as defined above may also be formulated to be especially suitable for vaginal or perivaginal administration. Suitable dosage forms to this end may include vaginal suppositories, creams, ointments, liquid formulations, pessaries, tampons, gels, pastes, foams or sprays. The suppository, cream, ointment, liquid formulation, pessary, tampon, gel, paste, foam or spray for vaginal or perivaginal delivery comprises a therapeutically effective amount of the selected active agent(s) and one or more conventional nontoxic carriers suitable for vaginal or perivaginal drug administration. The vaginal or perivaginal forms of the present invention may be manufactured using conventional processes as for example disclosed in Remington: The Science and Practice of Pharmacy, supra. The vaginal or perivaginal dosage unit may be fabricated to disintegrate rapidly or over a period of several hours. The time period for complete disintegration may be in the range of from about 10 minutes to about 6 hours, e.g., less than about 3 hours.

### e) Formulation suitable for topical formulations

A composition comprising Vitamin D and the agent as defined above may also be formulated to be especially suitable for topical administration. Suitable dosage forms to this end may include any form suitable for application to the body surface, and may comprise, for example, an ointment, cream, gel, lotion, solution, paste or the like, and/or may be prepared so as to contain liposomes, micelles, and/or microspheres. In certain embodiments, topical formulations herein are ointments, creams and gels.

### f) Formulation suitable for transdermal administration

A composition comprising Vitamin D and the agent as defined above may also be formulated to be especially suitable for transdermal administration. As known to one skilled in the art, transdermal administration involves the delivery of compounds via percutaneous passage of the compound into the systemic circulation of the patient. This can be affected e.g. by transdermal patches or iontophoresis devices. Other components besides Vitamin D and the agent as defined above may be incorporated into the transdermal patches as well. For example, compositions and/or transdermal patches may be formulated with one or more preservatives or bacteriostatic agents including, but not limited to, methyl hydroxybenzoate, propyl hydroxybenzoate, chlorocresol, benzalkonium chloride, and the like. Dosage forms of the inventive composition for topical administration of the Vitamin D and the agent as defined above may include creams, sprays, lotions, gels, ointments, eye drops, nose drops, ear drops, and the like. In such dosage forms, the ingredients of the inventive composition may be mixed to form a white, smooth, homogeneous, opaque cream or lotion with, for example, benzyl alcohol 1% or 2% (wt/wt) as a preservative, emulsifying wax, glycerin, isopropyl palmitate, lactic acid, purified water and sorbitol solution. In addition, the compositions may contain polyethylene glycol 400. They may be mixed to form ointments with, for example, benzyl alcohol 2% (wt/wt) as preservative, white petrolatum, emulsifying wax, and tenox II (butylated hydroxyanisole, propyl gallate, citric acid, propylene glycol). Woven pads or rolls of bandaging material, e.g., gauze, may be impregnated with the compositions in solution, lotion, cream, ointment or other such form may also be used for topical application. The compositions may also be applied topically using a transdermal system, such as one of an acrylic-based polymer adhesive with a resinous crosslinking agent impregnated with the composition and laminated to an impermeable backing.

Examples of suitable skin contact adhesive materials include, but are not limited to, polyethylenes, polysiloxanes, polyisobutylenes, polyacrylates, polyurethanes, and the like. Alternatively, the drug-containing reservoir and skin contact adhesive are separate and distinct layers, with the adhesive underlying the reservoir that, in this case, may be either a polymeric matrix as described above, or be a liquid or hydrogel reservoir, or take some other form.

### g) Formulation suitable for intravesical administration

The term intravesical administration is used herein in its conventional sense to mean delivery of a drug directly into the bladder. Suitable methods for intravesical administration may be found, for example, in US Patent Nos. 6,207,180 and 6,039,967.

Vitamin D and the agent as defined above may also be administered according to the medical uses and methods of treatment described herein in the form of a supplement, a food or drink product comprising Vitamin D and the agent as defined above.

It is to be understood that the uses herein described need not fulfill any regulatory requirement, e.g. for the definition of a drug or of a supplement. Preferably, the uses herein described are non-therapeutic.

As used herein, the term "supplement", or more in general "composition", refers to a composition preferably intended to supply any one or more of the above-defined ingredients to a subject that is missing such ingredients from his diet or that is otherwise deficient in such ingredients and/or to induce a beneficial health effect, for instance by preventing, controlling and/or treating any of the above-defined pathologies, disorders or symptoms. Preferably, supplements are formulated so as to be administered by mouth, e.g. as a pill, capsule, tablet, or liquid as described above.

As used herein, the term "food product" refers to a composition in the form of an ingestible substance which, at the temperature it is properly stored and ingested, is in a solid or semi solid form, and which will be chewed prior to swallowing. As used herein, the term "drink product" refers to a composition in the form of an ingestible substance which, at the temperature it is properly stored and ingested, is in a free-flowing liquid form, and which will not be chewed prior to swallowing.

The food product may in principle be any food product which has been processed to comprise Vitamin D and the agent as defined above to be administered. In the event Vitamin D and the agent as defined above is to be administered, the food product will comprise the relevant Vitamin D and the agent as defined above in the intended concentration. Examples of such food products include a food bar, such as a chocolate bar, granola bar, ice cream bar or energy bar; chewing gum; candy; a breath mint; yogurt; an edible gel; a ready-made meal, for example a freeze-dried ready-made meal; a spread; a pudding; or a processed fruit product such as a fruit rollup or a fruit stick. Examples of such drink products include fruit juice or fruit juice-containing drinks, dairy product drinks, e.g. milk-containing drinks or buttermilk-containing drinks, whey-containing drinks and yogurt-containing drinks, energy drinks, soft drinks, flavored water drinks etc.

The author has found that provision of a certain minimum amount of Vitamin D and the agent as defined above in the inventive composition can be advantageous in ensuring that the desired therapeutic or prophylactic effect can be achieved in a reasonable number of administrations. Typically, successful treatment or prevention of fibroids can be achieved by a daily administration of a total of as much as about 300 mg total EGCG, and about 50 µg total vitamin D. However, from the standpoint of the impact of any such repeated administration on patient quality of life, it can be advantageous to ensure a minimum amount of vitamin D and the agent as defined above in the composition so as to correspondingly decrease the number of daily administrations needed to reach a given target amount of such Vitamin D and the agent as defined above e.g. the target amount of Vitamin D and the agent as defined above indicated above.

Therefore, the medical uses and treatment methods set out herein may involve administering in one combined administration (e.g. in a tablet or in the form of a powder, e.g. provided in a sachet) to the subject as much as: about 300 mg of agent as defined above and 50 µg of vitamin D, about 150 mg of agent as defined above and 25 µg Vitamin D, about 100 mg of agent as defined above and 20 µg Vitamin D, about 75 mg of agent as defined above and 17.5 µg Vitamin D, about 50 mg of agent as defined above and 10 µg Vitamin D, 41.25 mg of agent as defined above and 6.9 µg Vitamin D, about 5 mg of agent as defined above and 1 µg Vitamin D.

One embodiment of the medical uses and treatment methods set out herein may also involve administering about 300 mg of agent as defined above and 50 µg of vitamin D, or between 150 and 300 mg of agent as defined above and 25 - 50 µg of vitamin D in any one administration, e.g. in any one combined administration, to the subject. One embodiment of the medical uses and treatment methods set out herein may involve administering between 5 and 1000 mg of the agent as defined above and 1 - 100 µg of vitamin D in any one administration, e.g. in any one combined administration, to the subject. Another embodiment of the medical uses and treatment methods set out herein may involve administering about 41.25 mg of the agent as defined above and 6.9 µg Vitamin D in any one administration, e.g. in any one combined administration, to the subject.

In one embodiment, if the Vitamin D and the agent as defined above is to be provided for administration to the subject in powder form, e.g. in a sachet, it may comprise or consist of about 150 mg of the agent as defined above and 25 µg of vitamin D, and this may be administered to the subject. In a related embodiment, the Vitamin D and the agent as defined above may be provided for administration to the subject in powder form, e.g. in a sachet, and the powder composition may comprise about 300 mg of the agent as defined above and 50 µg of Vitamin D, and this may be administered to the subject. Alternatively, in one embodiment, if Vitamin D and the agent as defined above is to be provided for administration to the subject in the form of a soft gel capsule, it may comprise or consist of about 150 mg of the agent as defined above and 25 µg of vitamin D, and this may be administered to the subject. In a related embodiment, the combination may be provided for administration to the subject in the form of a soft gel capsule, and the combination in the soft gel capsule may comprise 41.25 mg of the agent as defined above and 6.9 µg Vitamin D, and this may be administered to the subject.

Especially the latter embodiments comprising administering about 150 mg of the agent as defined above and 25 µg Vitamin D, are especially advantageous because to reach a daily target amount with a single dose.

The following amounts and combinations represent preferred embodiments of the present invention:
- about 5-1000 mg the agent as defined above and 1-100 µg Vitamin D;
- about 10-500 mg the agent as defined above and 5-70 µg Vitamin D;
- about 50-150 mg the agent as defined above and 10-60 µg Vitamin D;
- about 100-300 mg the agent as defined above and 25-50 µg Vitamin D;
- about 120-180 mg the agent as defined above, in particular 150 mg EGCG ± 20%;
- about 25 µg Vitamin D;
- about 150 mg of the agent as defined above and 25 µg Vitamin D;
- about 41.25 mg of the agent as defined above and 6.9 µg Vitamin D;
- about 82.5 mg the agent as defined above and about 13.8 µg Vitamin D;
- about 150 mg of the agent as defined above, 5 mg Vitamin B6 and 25 µg Vitamin D;
- vitamin D and the agent as defined above in a weight ratio of about 1 : 6000;
- vitamin D and the agent as defined above in a weight ratio of from about 1 : 50 - 1 : 1000000;
- about 150 mg the agent as defined above and 60 µg Vitamin D;
- vitamin D and the agent as defined above in a weight ratio of about 1 : 2500;
- about 300 mg the agent as defined above and 100 µg Vitamin D;
- vitamin D and the agent as defined above in a weight ratio of about 1 : 3000;
- vitamin D and the agent as defined above in a weight ratio of about 1 : 50;
- vitamin D and the agent as defined above in a weight ratio of about 1 : 1000000;
- vitamin D and the agent as defined above in a weight ratio of from about 1 : 2000 to 1 : 7000;
- Vitamin D and the agent as defined above in a weight ratio of from about 1 : 100-1 : 100000;
- vitamin D, vitamin B6 and the agent as defined above in a weight ratio of about 1 : 200 : 6000.

The following provides several examples illustrating various embodiments of the present invention, and the technical effects and advantages which it achieves. It should be understood that the following examples are presented for illustrative purposes only, and do not limit the claimed invention.

### Examples

The inventors have found that administration of Vitamin D and an agent such as EGCG can prevent/control/treat fibroids according to the present invention, particularly uterine fibroids, caused for instance by familiarity, age, ethnicity, obesity, hormonal activity, etc. This represents a new and unexpected approach to the prevention/control/treatment of fibroids.

### Example 1: Composition 1

A first exemplary composition of the present invention is in the form of a round tablet (diameter 10 mm), weighing about 500 mg, with an expected shelf life of 24 months, and having the following composition:

**Table 1. Composition 1**

| **Ingredient** | **mg/tablet** | **Intake per tablet** |
|---|---|---|
| Green tea (Camellia Sinensis) Leaves extract 95% polyphenols 45% EGCG | 333.35 | 150 mg of EGCG |
| Microcrystalline cellulose | 89.37 | |
| Calcium phosphate | 45.00 | |
| Vitamin D3 (1.000 IU) | 11.11 | 25 µg |
| Vitamin B6 (pyridoxine chlorohydrate) | 6.02 | 5 mg |
| Magnesium stearate | 5.00 | |
| Silicon dioxide | 5.00 | |
| Total weight mg | 500 | |

### Example 2: Composition 2

A second exemplary composition of the present invention is in the form of a round tablet (diameter 10 mm), with an expected shelf life of 24 months, and has the following composition:

**Table 2. Composition 2**

| **Ingredient** | **mg/tablet** | **Intake per tablet** |
|---|---|---|
| Green tea leaves extract 45% in EGCG | 333.35 | 150 mg of EGCG |
| Vitamin B6 HCl | 7.8 | 5 mg |
| Vitamin D3 | 14.5 | 25 µg |
| Dicalcium phosphate | 40 | |
| Microcrystalline cellulose | 84.35 | |
| Magnesium stearate | 15 | |
| Silicon dioxide | 5 | |
| Total mg | 500 mg | |

It should be noted that in the present example, the tolerance range for the EGCG titre is 120-180 mg/tablet (150 ± 20%).

The amount of ingredient indicated in the column "mg/tablet" of Table 1 and 2 refers to the amount of ingredient used to prepare one tablet. The amount of ingredient indicated in the column "intake per tablet" of Table 1 and 2 refers to the amount of ingredient present in one tablet. The overdoses applied to vitamins B6 and D3 are linked to the following factors:
- starting title of the raw material;
- starting chemical form of the molecular substrate;
- loss of substance during the production process.

It is advisable to administer one or two tablets of Composition 1 or 2 per day.

### Example 3: In vitro evaluation of anti-fibrotic properties of epigallocatechin gallate and vitamin D3, alone or in combination

### Materials and Methods

Anti-fibrotic properties of epigallocatechin gallate (EGCG) and vitamin D3 (alone or in combination) have been preliminary tested in human normal fibroblasts in 2D cultures. Human normal fibroblasts used in this experiment are Primary cell cultures. To discard any effect on fibroblast viability, proliferation and apoptosis were also investigated. Fibroblasts culture: DMEM, 10% FBS, 1% L-Glu, 1% Pen/strep for 18 days; Fibroblasts were allowed to grow in the above fibroblast culture for 18 days until reaching 50% confluence. Then, the fibrotic process was fostered by adding 10ng/ml TGF-β1 (Peprotech, cat no. #100-21-10UG). 24 hours after addition of TGF-β1, fibroblasts were thoroughly washed with PBS, fresh fibroblast culture medium and the below formulas were added. Fibroblasts were then allowed to grow for an additional 24 hours.

### Formulas as follows:

- Vitamin D3 (cholecalciferol, CAS-No. 67-97-0): 0.005 µg/ml in DMSO. Vitamin D3 was dissolved in DMSO and after that further dilutions were made in PBS. Essentially, the cells receive the same "dose" of DMSO, this allows a clean comparison of the test. In this case, DMSO 1:10000 was used;
- EGCG (GREEN TEA DRY EXTRACT 95% POLYPHENOLS; CAS-No. 84650-60-2): 0.03 mg/ml in H₂O;
- combined treatment: EGCG 0.03 mg/ml + vitamin D3 0.005 µg/ml;
- control: vehicle only (DMSO);

Fibronectin and collagen release were both measured in western blot for assessing the anti-fibrotic effect. Results are reported as arbitrary OD-units. When the treatment has stopped, 24 hours after addition of the treatment, the cells were frozen at -80°C overnight. The next day protein extraction was performed and subsequently the western blot.

Statistical analysis was performed. Results are significant with respect to the single treatments.

### Results

### - Phase I of the study

Addition of vitamin D3 alone, EGCG alone or the combination of vitamin D3 and EGCG does not significantly modify neither fibroblasts proliferation rate, nor their viability (data not shown). Cell morphology does not significantly change among treated and control cells treated with TGF-β1.

In control fibroblasts, a mild increase in both fibronectin and collagen release has been noticed after 24 hours of culture in conventional medium (Fig. 1 and 2). This slight increase can be viewed as a non-specific reaction to culture environment.

In vitamin D3 treated cells, Vitamin D3 slightly increases fibronectin deposition, while significantly reducing collagen release (Fig. 1 and 2).

In EGCG treated cells, EGCG significantly reduces both fibronectin and collagen release (Fig. 1 and 2).

The association vitamin D3+EGCG significantly (compared to the single treatments) decreases both fibronectin and collagen release in cultured fibroblast (Fig. 1 and 2). The components contributing to the majority of the volume in fibroids are fibronectin and collagen, therefore the reduction of production and deposition of these two proteins may decrease the size of fibroids.

### - Phase II of the study

Fibronectin and collagen release from fibroblasts were analyzed at 24 hours after addition of EGCG or vitamin D3 in previously stimulated fibroblast, as described above. In order to establish a dose-response relationship, different concentrations (1-100 µg/L vitamin D3 in DMSO; 0.5-10 mg/L EGCG in H₂O) of both compounds were tested. These *in vitro* concentrations can be deemed representative of *in vivo* dosages of 1-100 µg per day of vitamin D and 5-1000 mg per day of EGCG.

Data are reported in Fig. 3 A-B and 4 A-B and expressed as fold-decrease of OD-Units in respect to the control value, which was obtained for fibroblasts after 24 hours of stimulation with TGF-β1 (e.g. a decrease of 50% between two measurements would be referred to a "half-fold change"). Fold change is a measure describing how much a quantity changes between an original and a subsequent measurement. It is defined as the ratio between the two quantities; for quantities A and B, then the fold change of B with respect to A is B/A.

Figure 3A shows that concentrations of vitamin D3 (in particular from 5 µg/L to 100 µg/L) have effect on the reduction of fibronectin. Figure 3B shows that concentrations of EGCG (in particular from 5 mg/L to 10 mg/L) have effect on the reduction of fibronectin.

Figure 4A shows that concentrations of vitamin D3 (in particular from 5 µg/L to 100 µg/L) have effect on the reduction of collagen. Figure 4B shows that concentrations of EGCG (in particular from 3 mg/L to 10 mg/L) have effect on the reduction of collagen. An additive effect and/or synergy were also observed by comparing Fig. 5 to Fig. 3 and Fig. 6 to Fig. 4.

### Example 4: Clinical study

### Patients and Methods

A clinical study was carried out between March and October 2019, at the Hospital of Orvieto. Informed consent after the explanation of the study purpose was given by all participants. Ethical principles of the Helsinki Declaration and the national laws were followed. Inclusion criteria: 18 years of age or older, in premenopausal stage, with at least one myoma ≥2 cm³ (intramural, subserosal and/or submucosal) detected by vaginal and abdominal ultrasound, with moderately severe myoma-related symptoms, and do not require treatment other than regular observation. Exclusion criteria: pregnant women or intended to become pregnant during the next four months, currently breastfeeding, with severe anemia or medical morbidity, eligible to surgery, elevated liver enzymes, treatment (within the past 3 months) of hormones (estrogen, progestin, oral contraceptives), corticosteroids, food supplements having possible hormonal effects, use of selective progesterone receptor modulators (sPRMs) or gonadotropin-releasing hormone (GnRH) analogues within the past 6 months. Patients were divided in two groups: one group (15 patients) treated daily by oral route with 25 µg vitamin D + 150 mg EGCG + 5 mg vitamin B6, twice a day, for 4 months. The second group (15 patients) received no treatment (control), for 4 months. The primary outcome was the change of myoma volume analyzed by transvaginal ultrasonography (TVU), and/or transabdominal ultrasonography with the Voluson^{™} E8 ultrasound (GE Healthcare). The secondary outcomes were the variation of the number of myomas, distress by bleeding during menstrual period, feeling pressure in the pelvic area, and feeling sense of fatigue as well as Quality of Life (QoL) and the severity of symptoms (SS). QoL and SS were measured as described in Spies et al., The UFS-QOL, a New Disease-Specific Symptom and Health-Related Quality of Life Questionnaire for Leiomyomata. The American College of Obstetricians and Gynecologists. VOL. 99, NO. 2, 2002. The higher the score, the better the QoL in relation to health. The lower the score, the lower the SS. The subjective experience of bleeding was indicated as heavy, medium and normal, through a self-administered bleeding assessment. A complete medical history was collected from all women; a careful physical examination, ultrasound instrumental evaluation and compilation of questionnaires were performed at baseline (T₀) and after 4 months (T₁). From all myomas identified, the total myoma volume was calculated through the Voluson^{™} E8 ultrasound (GE Healthcare).

### Statistical analysis

Statistical analysis was performed by using unpaired t-test (2018 GraphPad Software, La Jolla, CA, USA), when comparing two groups, with the results being expressed as mean ± standard error of the mean (SEM). Comparisons for repeated measures was assessed for intragroup analysis by one-way ANOVA: values are indicated as mean ± SEM.

Wilcoxon-Mann-Whitney test were used for the analyses of QoL and SS: values are indicated as median, 25^{th} and 75^{th} percentile. Statistical significance was accepted at the level of p-value ≤ 0.05.

### Results

30 women with myomas aged between 28 and 46 years old were enrolled. The clinical characteristics of patients by group at baseline (T₀) are illustrated in Table 3.

**Table 3. Clinical characteristics of patients by group at baseline.**

| | **Treated *T₀*** | **Control *T₀*** | ***p*-value** |
|---|---|---|---|
| | *Mean* ± *SEM* | *Mean* ± *SEM* | |
| *Age* | 37.27 ± 1.15 | 37.67 ± 1.71 | 0.8475 |
| *Height* | 165.73 ± 1.78 | 167.73 ± 1.09 | 0.3469 |
| *Weight* | 62.13 ± 2.21 | 64.00 ± 1.64 | 0.5023 |
| *BMI (kg*/*m²)* | 22.67 ± 0.64 | 22.72 ± 0.49 | 0.9542 |
| *N° myomas* | 23 | 21 | |
| *Mean n° myomas* | 1.53 ± 0.19 | 1.40 ± 0.19 | 0.6256 |
| *Volume myomas cm³* | 10.84 ± 1.16 | 10.17 ± 1.43 | 0.7188 |
| *SS* | 22.67 ± 1.76 | 27.13 ± 2.04 | 0.1086 |
| *QoL* | 91.60 ± 5.55 | 84.93 ± 6.13 | 0.4270 |

The baseline characteristics of patients in the two groups (Treated with Vitamin D + EGCG vs Control). The groups were well matched, with no significant difference. A p-value ≤0.05 was considered statistically significant. All data resulted non-significant. Abbreviations: BMI, body mass index; SS, severity of symptoms; QoL, quality of life; SEM, standard error of the mean.

At T₀ the two groups were comparable for all parameters. No dropouts were recorded in either group. No adverse effects ascribable to the treatment were observed throughout the study period. The total number of myomas in the treated and control group was 23 and 21, respectively. In the treated group the incidence of myomas intramural, subserosal and submucosal were 43.75%, 12.5%, 43.75% respectively. In the control group 47.4% were intramural, 10.5% were subserosal and 42.1% submucosal. A significant reduction was observed in the volume of myomas (from 10.84 ± 1.16 cm³ at baseline to 8.04 ± 0.85 cm³ after 4 months of treatment, ρ < 0.0001) in the treated group (Fig. 7), corresponding to a 34.7% reduction of the myoma volume. The reduction of volumes was independent to the type of myomas. In the control group, myomas volumes significantly fluctuated from 10.17 ± 1.43 cm³ at T₀, to 10.94 ± 1.50 cm³ after 4 month-period observation (*p* < 0.001) corresponding to a 6.9% increase of the myoma volume (Fig. 7). A trend towards significance is seen between the myoma volumes of treated *versus* controls at T₁ (p = 0.0930). The number of myomas did not change, from T₀ to T₁, either in the treated and control group, showing an unvaried mean ± SEM (1.53 ± 0.19 in the treated group and 1.40 ± 0.19 in the control group, with a not significant difference in between groups) (Table 3). In Table 4 the incidence of the main myoma-related symptoms expressed in percentage (%) at T₀ and T₁, in treated and control group is illustrated. Changes from T₀ to T₁ of QoL in the treated group showed an increase (31, median value), while the control group showed a little decrease (-1, median value) and there was a statistically significant difference of changes from T₀ to T₁ between the two groups (*p*<0.0001) (Fig. 9A). Changes from T₀ to T₁ of SS in the treated group showed a reduction (-7, median value, corresponding to a 47% reduction of SS), while the control group showed no variation (0, median value) and there was a statistically significant difference of changes from T₀ to T₁ between the two groups (*p*<0.0001) (Fig. 9B). Then, significant improvement in all QoL aspects and SS, including daily activities, sexual function, bleeding and pelvic pain, was found.

**Table 4. Incidence of number, type of myomas and correlated symptoms at visit 1 and visit 2.**

| | **Treated** | **Treated** | **Control** | **Control** |
|---|---|---|---|---|
| | ***T₀*** | ***T₁*** | ***T₀*** | ***T₁*** |
| | *n° patients (%)* | *n° patients (%)* | *n° patients (%)* | *n° patients (%)* |
| *Myomas intramural* | 7 (43.75%) | 7 (43.75%) | 9 (47.4%) | 9 (47.4%) |
| *Myomas subserosal* | 2 (12.5%) | 2 (12.5%) | 2 (10.5%) | 2 (10.5%) |
| *Myomas submucosal* | 7 (43.75%) | 7 (43.75%) | 8 (42.1%) | 8 (42.1%) |
| *Normal bleeding* | 3 (20 %) | 5 (33.3 %) | 3 (20 %) | 3 (20 %) |
| *Heavy bleeding* | 8 (53.3 %) | 0 (0 %) | 6 (40 %) | 6 (40 %) |
| *Medium bleeding* | 4 (26.7 %) | 10 (66.7 %) | 6 (40 %) | 6 (40 %) |
| *Fatigue* | 10 (66.7 %) | 3 (20 %) | 8 (53.3 %) | 8 (53.3 %) |
| *Pelvic pain* | 8 (53.3 %) | 1 (6.7 %) | 8 (53.3 %) | 8 (53.3 %) |

| | | | | |
|---|---|---|---|---|
| Incidence is expressed as the percentage (%) of subjects having the type of myoma or the correlated symptoms in each group at the two timepoints (*T₀*, baseline or *T₁*, after 4 months); n, number of subjects. | | | | |

In conclusion, the oral supplementation of vitamin D in combination with EGCG has a considerable impact on women's reproductive health. Indeed, this combination may avoid surgical treatments, allowing women to maintain fertility and prevent complications at the gynecological level. The present combination is appropriate to improve QoL and reduce SS. Furthermore, these findings indicate that vitamin D and EGCG could be used to treat myomas to prevent a possible increment of size, thus controlling the fibroid. Additionally, vitamin D and EGCG could be administered along with other pharmacological therapy, or as pre-treatment before surgery; this might help patients to reduce side effects and possible high myoma volumes that can complicate surgery and increase risks.

## Claims

1. Vitamin D in combination with one or more agent selected from the group consisting of:
epigallocatechin gallate (EGCG), catechin, gallocatechin and epicatechin gallate (ECG), preferably selected from the group consisting of: epigallocatechin-3-gallate, (-)-epigallocatechin-3-gallate, (-)-gallocatechin-3-O-gallate (GCG), (-)-gallocatechin-3-gallate, (+)-catechin (C), (-)-epicatechin (EC), (+)-gallocatechin (GC), (-)-epigallocatechin (EGC), (-)-epicatechin-3-gallate, (-)-catechin-3-gallate and (+)-catechin-3-gallate, for use in the prevention, control and/ or treatment of a fibroid selected from a uterine fibroid or a fibroadenoma.

2. Vitamin D and the one or more agent for use according to claim 1, wherein said vitamin D is in an amount of from 1 µg to 100 µg per day, and/or said one or more agent is in an amount of from 5 mg to 1000 mg per day and/or said vitamin D and said one or more agent are in a weight ratio of from 1 : 50 to 1 : 1000000, preferably said vitamin D is in an amount of approximately 50 µg per day, and/or said one or more agent is in an amount of approximately 300 mg per day and/or said vitamin D and said one or more agent are in a weight ratio of approximately 1 : 6000, 1 : 50, 1 : 2000, 1 : 2500, 1 : 3000, 1 : 5000, 1 : 7000, 1 : 10000, or 1 : 1000000.

3. Vitamin D and the one or more agent according to claim 1 or 2, for use with at least one further agent selected from the group consisting of: vitamin B, a polyphenol and DL-alpha-tocopherol, preferably said vitamin D, said one or more agent and said at least one further agent are in a weight ratio of from 1 : 5000 : 100 to 1 : 10000 : 1000, preferably said vitamin D, said one or more agent and said at least one further agent are in a weight ratio of approximately 1 : 6000 : 200, 1 : 5000 : 1000, 1 : 10000 : 100 or 1 : 10000 : 1000, preferably said at least one further agent is vitamin B6, preferably said vitamin B6 is in an amount of from 1 to 100 mg per day, preferably said at least one further agent is in an amount of approximately 1 to 10 mg per day, preferably said at least one further agent is in an amount of approximately 1 to 5 mg per day.

4. Vitamin D and the one or more agent for use according to any one of the preceding claims, wherein said one or more agent is epigallocatechin gallate (EGCG) and/or wherein said fibroid is a uterine fibroid.

5. Vitamin D and the one or more agent for use according to any one of the preceding claims, wherein said one or more agent is in the form of a tea plant leaf or of an extract of a tea plant leaf, preferably said tea plant leaf is a green tea plant leaf, preferably comprising said agent in an amount of from 30 wt% to 95 wt%, preferably approximately 30 wt%, 45 wt%, 50 wt% or 95 wt%.

6. Vitamin D and the one or more agent for use according to any one of the preceding claims, wherein said vitamin D and said one or more agent reduce the release of fibronectin and/or collagen.

7. Vitamin D and the one or more agent for use according to any one of the preceding claims, wherein said fibroid is a uterine fibroid and said prevention, control and/or treatment of a uterine fibroid comprises the prevention, control and/or treatment of at least one symptom of a uterine fibroid, said symptom being preferably selected from the group consisting of: menorrhagia, dysmenorrhea, intermenstrual bleeding, pelvic pain (acute or chronic), painful sexual intercourse, anemia, a compromised reproductive function, subfertility, infertility, early pregnancy loss, recurrent abortion, preterm delivery and a pregnancy complication, fatigue and a pressure symptom such as bloatedness, increased urinary frequency, bowel disturbance.

8. Vitamin D and the one or more agent for use according to any one of the preceding claims, wherein said vitamin D and said one or more agent are administered simultaneously and/or orally and/or twice a day.

9. A composition comprising:
- vitamin D,
- one or more agent selected from the group consisting of: epigallocatechin gallate (EGCG), catechin, gallocatechin and epicatechin gallate (ECG), preferably selected from epigallocatechin-3-gallate, (-)-epigallocatechin-3-gallate, (-)-gallocatechin-3-O-gallate (GCG), (-)-gallocatechin- 3-gallate, (+)-catechin (C), (-)-epicatechin (EC), (+)-gallocatechin (GC), (-)- epigallocatechin (EGC), (-)-epicatechin-3-gallate, (-)-catechin-3-gallate and (+)-catechin-3-gallate,
- an excipient or a diluent, and
- optionally at least one further agent,
for use in the prevention, control and/or treatment of a fibroid selected from a uterine fibroid or a fibroadenoma, said one or more agent being preferably in the form as defined in claim 5, said excipient and/or diluent being preferably selected from the group consisting of: calcium phosphate, dicalcium phosphate, microcrystalline cellulose, magnesium stearate, silicon dioxide, sucrose, gum arabic, corn starch, medium-chain triglycerides, tricalcium phosphate, cross-linked sodium carboxymethylcellulose, hydroxypropyl methylcellulose, polyethylene glycol, titanium dioxide, polyvinylpyrrolidone, talc, erythritol, xylitol, steviol glycosides and sucralose, said at least one optional further agent being preferably selected from the group consisting of: vitamin B, a polyphenol and DL-alpha-tocopherol, preferably said composition is administered orally, or preferably said composition is in the form of a tablet, a hard capsule, a soft gel capsule, a powder, a syrup, a cachet, a troche, a lozenge or a pastille.

10. The composition for use according to claim 9, for use in combination with a further therapeutic intervention, said further therapeutic intervention being preferably selected from the group consisting of: surgery, a gonadotropin-releasing hormone agonist, a selective progesterone receptor modulator, an anti-hormonal agent, a hormone, an anti-inflammatory painkiller and tranexamic acid.

11. The composition for use according to claim 9 or 10, being a pharmaceutical composition, a supplement, a food product or a drink product.

12. The composition for use according to any one of claims 9 to 11, comprising: EGCG in the form of an extract of a green tea plant leaf, Microcrystalline cellulose, Calcium phosphate or Dicalcium phosphate, Vitamin D3, Vitamin B6, Magnesium stearate and Silicon dioxide.

## Patentansprüche

1. Vitamin D in Kombination mit einem oder mehreren Mitteln ausgewählt aus der Gruppe bestehend aus: Epigallocatechingallat (EGCG), Catechin, Gallocatechin und Epicatechingallat (ECG), vorzugsweise ausgewählt aus der Gruppe bestehend aus: Epigallocatechin-3-gallat, (-)-Epigallocatechin-3-gallat, (-)-Gallocatechin-3-O-gallat (GCG), (-)-Gallocatechin-3-gallat, (+)-Catechin (C), (-)-Epicatechin (EC), (+)-Gallocatechin (GC), (-)-Epigallocatechin (EGC), (-)-Epicatechin-3-gallat, (-)-Catechin-3-gallat und (+)-Catechin-3-gallat, zur Verwendung bei der Prävention, Kontrolle und/oder Behandlung eines Fibroms, ausgewählt aus einem Uterusmyom oder einem Fibroadenom.

2. Vitamin D und das eine oder die mehreren Mittel zur Verwendung nach Anspruch 1, wobei das Vitamin D in einer Menge von 1 µg bis 100 µg pro Tag vorliegt und/oder das eine oder die mehreren Mittel in einer Menge von 5 mg bis 1000 mg pro Tag vorliegen und/oder das Vitamin D und das eine oder mehrere Mittel in einem Gewichtsverhältnis von 1:50 bis 1:1000000 vorliegen, vorzugsweise liegt das Vitamin D in einer Menge von etwa 50 µg pro Tag vor und/oder das eine oder die mehreren Mittel liegen in einer Menge von etwa 300 mg pro Tag vor, und/oder das Vitamin D und das eine oder die mehreren Mittel liegen in einem Gewichtsverhältnis von etwa 1:6000, 1:50, 1:2000, 1:2500, 1:3000, 1:5000, 1:7000, 1:10000 oder 1:1000000 vor.

3. Vitamin D und das eine oder die mehreren Mittel nach Anspruch 1 oder 2, zur Verwendung mit mindestens einem weiteren Mittel, ausgewählt aus der Gruppe bestehend aus: Vitamin B, einem Polyphenol und DL-alpha-Tocopherol, wobei das Vitamin D, das eine oder die mehreren Mittel und das mindestens eine weitere Mittel vorzugsweise in einem Gewichtsverhältnis von 1:5000:100 bis 1:10000:1000 vorliegen, wobei das Vitamin D, das eine oder die mehreren Mittel und das mindestens eine weitere Mittel vorzugsweise in einem Gewichtsverhältnis von ungefähr 1:6000:200, 1:5000:1000, 1:10000:100 oder 1:10000:1000 vorliegen, wobei es sich bei dem mindestens einen weiteren Mittel vorzugsweise um Vitamin B6 handelt, vorzugsweise liegt das Vitamin B6 in einer Menge von 1 bis 100 vor mg pro Tag vor, vorzugsweise liegt das mindestens eine weitere Mittel in einer Menge von etwa 1 bis 10 mg pro Tag vor, vorzugsweise liegt das mindestens eine weitere Mittel in einer Menge von etwa 1 bis 5 mg pro Tag vor.

4. Vitamin D und das eine oder die mehreren Mittel zur Verwendung nach einem der vorhergehenden Ansprüche, wobei es sich bei dem einen oder den mehreren Mitteln um Epigallocatechingallat (EGCG) handelt und/oder wobei es sich bei dem Fibrom um ein Uterusmyom handelt.

5. Vitamin D und das eine oder die mehreren Mittel zur Verwendung nach einem der vorhergehenden Ansprüche, wobei das eine oder die mehreren Mittel in Form eines Teepflanzenblattes oder eines Extrakts eines Teepflanzenblattes vorliegen, vorzugsweise handelt es sich bei dem Teepflanzenblatt um ein Grüntee-Pflanzenblatt, vorzugsweise umfassend das Mittel in einer Menge von 30 Gew.-% bis 95 Gew.-%, vorzugsweise etwa 30 Gew.-%, 45 Gew.-%, 50 Gew.-% oder 95 Gew.-%.

6. Vitamin D und das eine oder die mehreren Mittel zur Verwendung nach einem der vorhergehenden Ansprüche, wobei das Vitamin D und das eine oder die mehreren Mittel die Freisetzung von Fibronektin und/oder Kollagen reduzieren.

7. Vitamin D und das eine oder die mehreren Mittel zur Verwendung nach einem der vorhergehenden Ansprüche, wobei es sich bei dem Fibrom um ein Uterusmyom handelt und die Prävention, Kontrolle und/oder Behandlung eines Uterusmyoms die Prävention, Kontrolle und/oder Behandlung von mindestens einem Symptom eines Uterusmyoms umfasst, wobei das Symptom vorzugsweise ausgewählt ist aus der Gruppe bestehend aus: Menorrhagie, Dysmenorrhoe, intermenstrueller Blutung, Beckenschmerzen (akut oder chronisch), schmerzhaftem Geschlechtsverkehr, Anämie, einer beeinträchtigten Fortpflanzungsfunktion, Subfertilität, Infertilität, frühem Schwangerschaftsverlust, wiederholten Fehlgeburten, Frühgeburt und Schwangerschaftskomplikation, Müdigkeit und einem Drucksymptom wie Aufgeblähtheit, erhöhter Harndrangfrequenz, Darmstörung.

8. Vitamin D und das eine oder die mehreren Mittel zur Verwendung nach einem der vorhergehenden Ansprüche, wobei das Vitamin D und das eine oder die mehreren Mittel gleichzeitig und/oder oral und/oder zweimal täglich verabreicht werden.

9. Zusammensetzung umfassend:
- Vitamin D,
- ein oder mehrere Mittel, ausgewählt aus der Gruppe bestehend aus: Epigallocatechingallat (EGCG), Catechin, Gallocatechin und Epicatechingallat (ECG), vorzugsweise ausgewählt aus Epigallocatechin-3-gallat, (-)-Epigallocatechin-3-gallat, (-)-Gallocatechin-3-O-gallat (GCG), ()-Gallocatechin-3-gallat, (+)-Catechin (C), (-)-Epicatechin (EC), (+)-Gallocatechin (GC), (-)-Epigallocatechin (EGC), (-)-Epicatechin-3-gallat, (-)-Catechin-3-gallat und (+)-Catechin-3-gallat,
- einen Hilfsstoff oder ein Verdünnungsmittel und
- optional mindestens ein weiteres Mittel,
zur Verwendung bei der Prävention, Kontrolle und/oder Behandlung eines Fibroms, ausgewählt aus einem Uterusmyom oder einem Fibroadenom, wobei das eine oder die mehreren Mittel vorzugsweise in der Form nach der Definition in Anspruch 5 vorliegen, wobei der Hilfsstoff und/oder das Verdünnungsmittel vorzugsweise ausgewählt sind aus der Gruppe bestehend aus: Calciumphosphat, Dicalciumphosphat, mikrokristalliner Cellulose, Magnesiumstearat, Siliciumdioxid, Saccharose, Gummi arabicum, Maisstärke, mittelkettigen Triglyceriden, Tricalciumphosphat, vernetzter Natriumcarboxymethylcellulose, Hydroxypropylmethylcellulose, Polyethylenglykol, Titandioxid, Polyvinylpyrrolidon, Talk, Erythritol, Xylitol, Steviolglykosiden und Sucralose, wobei das mindestens eine optionale weitere Mittel vorzugsweise aus der Gruppe ausgewählt ist, bestehend aus: Vitamin B, einem Polyphenol und DL-alpha-Tocopherol, wobei die Zusammensetzung vorzugsweise oral verabreicht wird, oder vorzugsweise liegt die Zusammensetzung in Form einer Tablette, einer Hartkapsel, einer Weichgelkapsel, eines Pulvers, eines Sirups, eines Cachets, einer Lutschpastille oder einer Pastille vor.

10. Zusammensetzung zur Verwendung nach Anspruch 9 zur Verwendung in Kombination mit einer weiteren therapeutischen Intervention, wobei die weitere therapeutische Intervention vorzugsweise ausgewählt ist aus der Gruppe bestehend aus: einer Operation, einem Gonadotropin-Releasing-Hormon-Agonisten, einem selektiven Progesteronrezeptor-Modulator, einem Anti-Hormonwirkstoff, einem Hormon, einem entzündungshemmenden Schmerzmittel und Tranexamsäure.

11. Zusammensetzung zur Verwendung nach Anspruch 9 oder 10, bei der es sich um eine pharmazeutische Zusammensetzung, ein Nahrungsergänzungsmittel, ein Lebensmittelprodukt oder ein Getränkeprodukt handelt.

12. Zusammensetzung zur Verwendung nach einem der Ansprüche 9 bis 11, umfassend: EGCG in Form eines Extrakts aus einem Grüntee-Pflanzenblatt, mikrokristalline Cellulose, Calciumphosphat oder Dicalciumphosphat, Vitamin D3, Vitamin B6, Magnesiumstearat und Siliciumdioxid.

## Revendications

1. Vitamine D en combinaison avec un ou plusieurs agents choisis dans le groupe constitué par :
le gallate d'épigallocatéchine (EGCG), la catéchine, la gallocatéchine et le gallate d'épicatéchine (ECG),
de préférence choisis dans le groupe constitué par :
l'épigallocatéchine-3-gallate, le (-)-épigallocatéchine-3-gallate, le (-)-gallocatéchine-3-O-gallate (GCG), le (-)-gallocatéchine-3-gallate, la (+)-catéchine (C), la (-)-épicatéchine (EC), la (+)-gallocatéchine (GC), la (-)-épigallocatéchine (EGC), le (-)-épicatéchine-3-gallate, le (-)-catéchine-3-gallate et le (+)-catéchine-3-gallate destiné à être utilisé dans la prévention, le contrôle et/ou le traitement d'un fibroïde choisi parmi un fibroïde utérin ou un fibroadénome.

2. Vitamine D et l'un ou plusieurs agents destinés à être utilisés selon la revendication 1, dans lesquels ladite vitamine D est en une quantité de 1 µg à 100 µg par jour et/ou lesdits un ou plusieurs agents sont en une quantité de 5 mg à 1 000 mg par jour et/ou ladite vitamine D et les dits un ou plusieurs agents sont dans un rapport en poids de 1 : 50 à 1 : 1 000 000, de préférence ladite vitamine D est en une quantité d'environ 50 □g par jour et/ou lesdits un ou plusieurs agents sont en une quantité d'environ 300 mg par jour et/ou ladite vitamine D et les un ou plusieurs agents sont dans un rapport en poids d'environ 1 : 6 000, 1 : 50, 1 : 2 000, 1 : 2 500, 1 : 3 000, 1 : 5 000, 1 : 7 000, 1 : 10 000 ou 1 : 1 000 000.

3. Vitamine D et l'un ou plusieurs agents selon la revendication 1 ou 2, destinés à être utilisés avec au moins un autre agent choisi dans le groupe constitué par : la vitamine B, un polyphénol et le DL-alpha-tocophérol, de préférence ladite vitamine D, lesdits un ou plusieurs agents et ledit au moins un autre agent sont dans un rapport en poids de 1 : 5 000 : 100 à 1 : 10 000 : 1 000, de préférence ladite vitamine D, lesdits un ou plusieurs agents et ledit au moins un autre agent sont dans un rapport en poids d'environ 1 : 6 000 : 200, 1 : 5 000 : 1 000, 1 : 10 000 : 100 ou 1 : 10 000 : 1 000, de préférence ledit au moins autre agent est la vitamine B6, de préférence ladite vitamine B6 est en une quantité de 1 à 100 mg par jour, de préférence l'au moins un autre agent est en une quantité d'environ 1 à 10 mg par jour, de préférence ledit au moins un autre agent est en une quantité de 1 à 5 mg par jour.

4. Vitamine D et l'un ou plusieurs agents destinés à être utilisés selon l'une quelconque des revendications précédentes, dans lesquels lesdits un ou plusieurs autres agents est le gallate d'épigallocatéchine (ECGC) et/ou ledit fibroïde étant un fibroïde utérin.

5. Vitamine D et l'un ou plusieurs agents destinés à être utilisés selon l'une quelconque des revendications précédentes, dans lesquels lesdits un ou plusieurs agents sont sous la forme d'une feuille de plante de thé ou d'un extrait d'une feuille de plante de thé, de préférence ladite feuille de plante de thé est une feuille de plante de thé vert, de préférence comprenant ledit agent en une quantité de 30 % en poids à 95 % en poids, de préférence environ 30 % en poids, 45 % en poids, 50 % en poids ou 95 % en poids.

6. Vitamine D et l'un ou plusieurs agents destinés à être utilisés selon l'une quelconque des revendications précédentes, dans lesquels ladite vitamine D et lesdits un ou plusieurs agents réduisent la libération de fibronectine et/ou de collagène.

7. Vitamine D et l'un ou plusieurs agents destinés à être utilisés selon l'une quelconque des revendications précédentes, dans lesquels ledit fibroïde est un fibroïde utérin et ladite prévention, contrôle et/ou traitement d'un fibroïde utérin comprend la prévention, le contrôle et/ou le traitement d'au moins un symptôme d'un fibroïde utérin, ledit symptôme étant de préférence choisi dans le groupe constitué par : la ménorrhagie, la dysménorrhée, le saignement intermenstruel, la douleur pelvique (aiguë ou chronique), l'intercours sexuel douloureux, l'anémie, une fonction reproductrice compromise, la sous-fertilité, l'infertilité, la perte de grossesse précoce, l'avortement récurrent, la délivrance à préterme et une complication de grossesse, la fatigue et un symptôme de pression tel que le ballonnement, une fréquence urinaire accrue, un trouble de l'intestin.

8. Vitamine D et l'un ou plusieurs agents destinés à être utilisés selon l'une quelconque des revendications précédentes, dans lesquels ladite vitamine D et lesdits un ou plusieurs agents sont administrés simultanément et/ou oralement et/ou deux fois par jour.

9. Composition comprenant :
- de la vitamine D,
- un ou plusieurs agents choisis dans le groupe constitué par : le gallate d'épigallocatéchine (EGCG), la catéchine, la gallocatéchine et le gallate d'épicatéchine (ECG), de préférence choisis parmi l'épigalocatéchine-3-gallate, le (-)-épigallocatéchine-3-gallate, le (-)-gallocatéchine-3-O-gallate (GCG), le (-)-gallocatéchine-3-gallate, la (+)-catéchine (C), la (-)-épicatéchine (EC), la (+)-gallocatéchine (GC), la (-)-épigallocatéchine (EGC), le (-)-épicatéchine-3-gallate, le (-)-catéchine-3-gallate et le (+)-catéchine-3-gallate,
- un excipient ou un diluant et
- éventuellement au moins un autre agent,
destinés à être utilisés dans la prévention, le contrôle et/ou le traitement d'un fibroïde choisi parmi un fibroïde utérin ou un fibroadénome, lesdits un ou plusieurs agents étant de préférence sous la forme telle que définie dans la revendication 5, ledit excipient et/ou diluant étant de préférence choisi dans le groupe constitué par : le phosphate de calcium, le phosphate de dicalcium, la cellulose microcristalline, le stéarate de magnésium, le dioxyde de silicium, le saccharose, la gomme arabique, l'amidon de maïs, les triglycérides à chaîne moyenne, le phosphate de tricalcium, la carboxyméthylcellulose de sodium réticulée, l'hydroxypropyl méthylcellulose, le polyéthylène glycol, le dioxyde de titane, la polyvinylpyrrolidone, le talc, l'érythritol, le xylitol, les glycosides de stéviol et le sucralose, ledit au moins un autre agent optionnel étant de préférence choisi dans le groupe constitué par : la vitamine B, un polyphénol et le DL-alpha-tocophérol, de préférence ladite composition est administrée oralement ou de préférence ladite composition est sous la forme d'un comprimé, d'une capsule dure, d'une capsule de gel mou, d'une poudre, d'un sirop, d'un cachet, d'une pastille, d'un losange ou d'une pastille.

10. Composition destinée à être utilisée selon la revendication 9, destinée à être utilisée en combinaison avec une autre intervention thérapeutique, ladite autre intervention thérapeutique étant de préférence choisie dans le groupe constitué par : une chirurgie, un agoniste d'hormone libérant la gonadotropine, un modulateur de récepteur de progestérones sélectif, un agent anti-hormonal, une hormone, un analgésique anti-inflammatoire et l'acide tranéxamique.

11. Composition destinée à être utilisée selon la revendication 9 ou 10, étant une composition pharmaceutique, un complément, un produit alimentaire ou un produit de boisson.

12. Composition destinée à être utilisée selon l'une quelconque des revendications 9 à 11, comprenant : de l'EGCG sous la forme d'un extrait de feuille de plante de thé vert, de cellulose microcristalline, de phosphate de calcium ou de phosphate de dicalcium, de vitamine D3, de vitamine B6, de stéarate de magnésium et de dioxyde de silicium.
